# EUROPEAN PATENT APPLICATION

(11) **EP 4 520 433 A1**
(43) Date of publication of application: **12.03.2025**
(21) Application number: 23799724.2
(22) Date of filing: 04.05.2023
(51) Int. Cl.: B01L 7/00, B01L 3/00

(54) **NUCLEIC ACID DETECTION CARTRIDGE**

(30) Priority: 06.05.2022 KR 20220056173; 06.05.2022 KR 20220056174; 06.05.2022 KR 20220056175; 06.05.2022 KR 20220056176
(71) Applicant: Seegene, Inc., Seoul 05548 (KR)
(72) Inventor: JIN, Young Joon, Seoul 08613 (KR); LEE, Gi-Hun, Incheon 22101 (KR); KANG, Jin Seok, Yongin-si, Gyeonggi-do 16950 (KR); SONG, Young Ho, Bucheon-si, Gyeonggi-do 14786 (KR); PARK, Ji Hoon, Hanam-si, Gyeonggi-do 12942 (KR)
(74) Representative: Beck & Rössig European Patent Attorneys
(86) International application number: PCT/KR2023/006161
(87) International publication number: WO 2023/214843

(57) **Abstract**

A nucleic acid detection cartridge according to the present invention comprises: a chamber body including a plurality of chambers provided on the upper surface; and a cover facing the upper surface of the chamber body, wherein at least one of the chamber body and the cover can move relative to the other in a plane direction parallel to the upper surface, and at least one of the chamber body and the cover cam move relative to the other in a straight line a certain angle with respect to the upper surface.

## Description

### TECHNICAL FIELD

The present disclosure relates to a nucleic acid detection cartridge including a cover.

### BACKGROUND OF THE INVENTION

As interest in health among modern individuals increases and life expectancy extends, the importance of accurate analysis of pathogens and in vitro nucleic acid-based molecular diagnosis, such as genetic analysis for patients, is rising, leading to an increased demand. Nucleic acid-based molecular diagnosis involves extracting nucleic acids from a sample and then confirming the presence or absence of a target nucleic acid among the extracted nucleic acids.

Polymerase chain reaction (PCR) is the most widely used nucleic acid amplification method, encompassing repeated cycles of denaturation of double-stranded deoxyribonucleic acid (DNA), annealing of oligonucleotide primers to the DNA templates, and extension of the primers by DNA polymerase (Mullis et al.; U.S. Pat. Nos. 4,683,195, 4,683,202 and 4,800,159; Saiki et al., (1985) Science 230, 1350-1354).

Real-time PCR using a fluorescent material is a method of detecting an increase in fluorescence intensity according to nucleic acid amplification during a PCR process. Real-time PCR enables multiplex detection by using different fluorescent dyes for respective targets, however, it requires expensive equipment and a considerable period of time for detection.

Meanwhile, recent point-of-care (POC) diagnostic techniques, which enable accurate and rapid diagnosis of diseases without being restricted by time or location, are gaining attention as crucial techniques for evidence-based precision medicine.

However, POC diagnostic devices with a simplified structure, a compact size, and a reduced processing time often face the issue of low detection accuracy for target substances.

### SUMMARY OF THE INVENTION

### TECHNICAL PROBLEM

i) Against this backdrop, an embodiment of the present disclosure provides a nucleic acid detection cartridge having a cover assembly whose position may be changed relative to a chamber body.
ii) According to another aspect, an embodiment of the present disclosure provides a target analyte detection cartridge capable of sample filtering that enables unrefined crude samples to be directly injected into the cartridge for use.
iii) According to another aspect, an embodiment of the present disclosure provides a target analyte detection cartridge having a cover assembly capable of sealing a detection well.
iv) According to another aspect, an embodiment of the present disclosure provides a target analyte detection cartridge capable of rapid and accurate detection through arrangement of detection chambers.

### TECHNICAL SOLUTION

i) In order to achieve the above objectives, a first embodiment of the present disclosure may provide a nucleic acid detection cartridge, including a chamber body including a plurality of chambers provided on an upper surface thereof, and a cover facing the upper surface of the chamber body, wherein at least one of the chamber body and the cover is provided to be movable relative to each other on a plane that is parallel to the upper surface, and at least one of the chamber body and the cover is provided to be movable relative to each other in a direction that is perpendicular to the upper surface.

Here, the plurality of chambers include chambers arranged in a circumferential direction with respect to a center of the chamber body, and the cover is provided to cover the chambers.

Here, at least one of the chamber body and the cover is provided to be rotationally movable relative to each other about an axis of rotation, and at least one of the chamber body and the cover is provided to be able to translate relative to each other in a direction of the axis of rotation.

Here, the cover includes a pipette hole provided on a movement path of a pipette or a pipette tip that aspirates fluids from one or more of the plurality of chambers and injects fluids into one or more of the plurality of chambers.

Here, the nucleic acid detection cartridge further includes the pipette tip coupled to an end portion of the pipette, and provided to be vertically movable through the pipette hole of the cover.

Here, the cover has a pipette tip guide that extends upward from the pipette hole and accommodates the pipette tip therein.

Here, the pipette tip includes a connecting portion to which the pipette is able to be coupled, a pipette tip body extending downward from the connecting portion to form a flow path, a tip portion extending downward from the pipette tip body, and a pipette tip filter member provided in the flow path inside the pipette tip body.

Here, the chamber body rotates about a central axis, and the plurality of chambers include chambers arranged in the circumferential direction with respect to a center of the chamber body.

Here, the chamber body is provided in a shape of a circular disk or a shape of a portion of a disk, and the cover includes an inner side surface facing an outer side surface of the chamber body, and an upper portion facing the upper surface of the chamber body.

Here, the nucleic acid detection cartridge further includes a sealing ring that is provided on the inner side surface of the cover or on the outer side surface of the chamber body, and blocks air outside the cover.

Here, the sealing ring is provided in a lower portion of the outer side surface of the chamber body, when the cover is relatively distant from the chamber body in the direction of the axis of rotation, the sealing ring is not in contact with the cover, and when the cover is relatively close to the chamber body in the direction of the axis of rotation, the sealing ring comes into contact with the cover.

Here, the sealing ring has a coefficient of friction that allows relative rotation of the cover and the chamber body.

Here, the nucleic acid detection cartridge further includes a sealing film that is attached or coupled to the upper surface of the chamber body to seal the plurality of chambers.

Here, the pipette tip is able to descend to penetrate the sealing film.

Here, vertical guide structures are provided on the inner side surface of the cover and the outer side surface of the chamber body, respectively, to guide a vertical movement of the chamber body or the cover, and the vertical guide structures allow the chamber body or the cover to move in the direction of the axis of rotation at a predetermined position in the circumferential direction.

Here, the nucleic acid detection cartridge further includes vertical guide structures that guide a vertical movement of the chamber body or the cover, wherein the vertical guide structures are provided on the upper surface of the chamber body and a bottom surface of the cover that faces the upper surface of the chamber body, respectively, and the vertical guide structures allow the chamber body or the cover to move in the direction of the axis of rotation at a predetermined position in the circumferential direction.

Here, the chamber body further includes a sample accommodation chamber into which a sample is injected, and the cover includes a sample injection hole capable of, when aligned with the sample accommodation chamber, opening the sample accommodation chamber.

Here, the sample accommodation chamber is provided to be spatially partitioned into a sample injection region where the sample is injected, and a sample withdrawal region where the sample is withdrawn, and the sample accommodation chamber has a filter member through which the sample injected into the sample injection region passes before being withdrawn from the sample withdrawal region.

Here, the sample injection hole is positioned above the sample injection region when the sample is injected, and is positioned above the sample withdrawal region when a nucleic acid is detected.

Here, the cover has a sample guide portion that extends toward a lower side of the sample injection hole and is able to be accommodated in the sample withdrawal region as the chamber body and the cover come into proximity to each other.

Here, the chamber body further includes a detection chamber in which a nucleic acid is detected, and the cover has a light-transmitting portion capable of transmitting detection light into the detection chamber.

Here, the detection chamber has a detection well in which the nucleic acid is detected, the cover has a detection well sealing portion capable of sealing the detection well, and at least one of the chamber body and the cover is able to be selectively positioned relative to each other in a first position where the detection well sealing portion opens the detection well, and a second position where the detection well sealing portion seals the detection well.

In addition, there may be provided a nucleic acid detection cartridge, including a chamber body including a sample accommodation chamber into which a sample is injected, and a detection chamber in which a detection well in which a nucleic acid is detected is provided, and a cover having an opening capable of opening the sample accommodation chamber, and a light-transmitting portion capable of transmitting detection light into the detection well, wherein at least one of the chamber body and the cover is provided to be selectively positioned relative to each other in a first position where the sample is withdrawn from the sample accommodation chamber through the opening, and a second position where the nucleic acid is detected in the detection well, and at least one of the chamber body and the cover is provided to be closer to the second position than the first position, relative to each other.

In addition, there may be provided a nucleic acid detection cartridge, including a chamber body having a plurality of chambers provided on an upper surface thereof, a cover facing the upper surface of the chamber body, and a pipette tip configured to be vertically movable relative to the cover, and to be coupled to an end portion of a pipette, wherein at least one of the chamber body and the cover is provided to be rotatable relative to each other, and the pipette tip transfers fluids between the plurality of chambers while changing a relative position thereof in a circumferential direction of the chamber body together with the cover.

Here, the cover includes a pipette hole provided on a movement path of the pipette tip.

Here, the cover has a pipette tip guide that extends upward from the pipette hole and accommodates the pipette tip therein.

Here, the pipette tip includes a connecting portion to which the pipette is able to be coupled, a pipette tip body extending downward from the connecting portion to form a flow path, a tip portion extending downward from the pipette tip body, and a pipette tip filter member provided in the flow path inside the pipette tip body.

Here, the connecting portion has a first catching protrusion that extends toward an outside of an opening of the pipette tip guide such that the pipette tip is seated on the pipette tip guide.

Here, the pipette tip body has a second catching protrusion that extends toward an outside of an opening of the pipette tip guide to prevent the pipette tip from being separated upward from the cover.

Here, the second catching protrusion is provided to protrude in a circumferential direction of the pipette tip body, and to be elastically deformable.

Here, the pipette tip guide includes a step structure in which an upper inner diameter is less than a lower inner diameter, and the second catching protrusion has an outer diameter greater than the upper inner diameter of the pipette tip guide.

Here, the nucleic acid detection cartridge further includes a sealing film that is attached or coupled to the upper surface of the chamber body to seal the plurality of chambers.

Here, the sealing film is configured to be penetrated by the pipette tip.

Here, the plurality of chambers include chambers arranged in the circumferential direction with respect to a center of the chamber body, and the cover is provided to cover the chambers.

Here, at least one of the chamber body and the cover is provided to be rotationally movable relative to each other.

Here, at least one of the chamber body and the cover is provided to be able to translate relative to each other in a direction of the axis of rotation.

Here, the chamber body has a plurality of chambers arranged in the circumferential direction with respect to a center, and detection chambers arranged side by side, and the detection chambers are arranged such that a straight line having a certain angle to any one radial direction of the chamber body passes through the detection chambers.

Here, the detection chambers are arranged such that the pipette performing a relative circular motion at a certain radius away from a central axis of the chamber body passes through the detection chambers.

Here, the detection chamber includes a detection well in which a nucleic acid is detected, and an inclined portion that guides a sample to the detection well.

Here, the detection chamber is provided in a form of an elongated hole extending in a direction parallel to the radial direction, and the detection well is positioned farther away in the radial direction than the inclined portion.

Here, the cover has a detection well sealing portion capable of sealing the detection well, and at least one of the chamber body and the cover is able to be selectively positioned relative to each other in a first position where the detection well sealing portion opens the detection well, and a second position where the detection well sealing portion seals the detection well.

Here, the cover has a light-transmitting portion capable of transmitting detection light into the detection well.

Here, the chamber body further includes a sample accommodation chamber into which a sample is injected, the sample accommodation chamber includes a sample injection region into which the sample is injected, a sample withdrawal region that is spatially separated from the sample injection region, and from which the sample is withdrawn, and a filter member, and the sample injected into the sample injection region passes through the filter member in a direction opposite to gravity, and then moves to the sample withdrawal region.

Here, the chamber body includes the plurality of chambers including a sample accommodation chamber in which a sample is accommodated, and detection chambers each having a detection well in which a nucleic acid is detected, the cover includes a detection well sealing portion capable of sealing the detection well, and at least one of the chamber body and the cover is able to be selectively positioned relative to each other in a first position where the detection well sealing portion opens the corresponding detection well, and a second position where the detection well sealing portion seals the corresponding detection well.

Here, the chamber body is provided in a shape of a circular disk or a shape of a portion of a disk, and includes the plurality of chambers that are arranged in a circumferential direction with respect to a central axis, and include a sample accommodation chamber, and detection chambers in which nucleic acids are detected, and the detection chambers are arranged side by side such that a straight line having a certain angle to any one radial direction of the chamber body passes through the detection chambers.

ii) In order to achieve the above objectives, a second embodiment of the present disclosure may provide a nucleic acid detection cartridge, including a chamber body including a sample accommodation chamber in which a sample is accommodated, wherein the sample accommodation chamber includes a sample injection region into which the sample is injected, a sample withdrawal region that is spatially separated from the sample injection region, and from which the sample is withdrawn, and a filter member, and the sample injected into the sample injection region passes through the filter member in a direction opposite to gravity, and then moves to the sample withdrawal region.

Here, the nucleic acid detection cartridge further includes a cover having a sample injection hole that faces the upper surface of the chamber body and is able to open the sample injection region when aligned with the sample accommodation chamber, and at least one of the chamber body and the cover is provided to be movable relative to each other on a plane parallel to the plane with which they face each other.

Here, the chamber body is provided on the upper surface and includes a plurality of chambers arranged in a circumferential direction with respect to the center of the chamber body, and the cover is provided to cover the chambers.

Here, at least one of the chamber body and the cover is provided to be rotationally movable relative to each other about an axis of rotation, and at least one of the chamber body and the cover is provided to be able to translate relative to each other in a direction of the axis of rotation.

Here, the sample accommodation chamber includes a portion having a lower cross-sectional area that is smaller than an upper cross-sectional area.

Here, the sample injection region has an inclined surface that guides the sample to the sample withdrawal region.

Here, the sample injection region has a step in which the cross-sectional area discontinuously decreases toward the bottom.

Here, the volume of an upper region of the step is provided to be at least twice the volume of a lower region of the step.

Here, the sample withdrawal region has an inclined surface provided such that the cross-sectional area decreases toward the bottom.

Here, the sample accommodation chamber includes a blocking portion that partitions the sample injection region and the sample withdrawal region, and a sample flow channel that allows sample flow between the sample injection region and the sample withdrawal region.

Here, the blocking portion is provided as a blocking wall surrounding the sample withdrawal region, and the sample flow channel is configured to penetrate the blocking wall.

Here, the sample flow channel is configured to penetrate both facing sides of the blocking wall.

Here, the blocking portion is detachably coupled to the bottom surface of the sample accommodation chamber, and the sample flow channel is configured as a groove that is recessed upward from a lower end of the blocking portion.

Here, the filter member is spaced apart from the bottom surface of the sample accommodation chamber and installed to vertically divide the internal space defining the sample withdrawal region.

Here, the filter member is provided to be higher than the sample flow channel and installed to vertically divide an internal space partitioned by the blocking wall.

Here, the filter member is provided to be separable from the blocking wall.

Here, the chamber body further includes a detection chamber in which a nucleic acid is detected, the cover has a light-transmitting portion capable of transmitting detection light into the detection chamber, the sample injection hole is positioned above the sample injection region when the sample is injected, and is positioned above the sample withdrawal region when a nucleic acid is detected, and the light-transmitting portion is positioned above the detection chamber.

Here, the cover further includes a sample guide portion extending toward the bottom of the sample injection hole.

Here, the sample accommodation chamber further includes a blocking wall surrounding the sample withdrawal region, and the sample guide portion is provided to overlap with the blocking wall when at least one of the chamber body and the cover is relatively close to each other in the direction of the axis of rotation.

iii) In order to achieve the above objectives, a third embodiment of the present disclosure may provide a nucleic acid detection cartridge, including a chamber body including a sample accommodation chamber in which a sample is accommodated, and detection chambers each having a detection well in which a nucleic acid is detected, and a cover that is provided to cover an upper surface of the chamber body, and has a detection well sealing portion capable of sealing the detection well, wherein at least one of the chamber body and the cover is able to be selectively positioned relative to each other in a first position where the detection well sealing portion opens the corresponding detection well, and a second position where the detection well sealing portion seals the corresponding detection well.

Here, at least one of the chamber body and the cover is provided to be movable relative to each other in a direction perpendicular to the upper surface.

Here, at least one of the chamber body and the cover is provided to be movable relative to each other on a plane parallel to the upper surface.

Here, at least one of the chamber body and the cover is provided to be rotationally movable relative to each other, and at least one of the chamber body and the cover is provided to be able to translate relative to each other in a direction of the axis of rotation.

Here, the chamber body may be selectively positioned in the first position and the second position while rotating.

Here, the detection well sealing portion is provided as a plug inserted into an inlet of the detection well.

Here, the detection well sealing portion is provided to seal the detection well as at least one of the chamber body and the cover comes into proximity to each other in the direction of the axis of rotation.

Here, the detection well sealing portion is provided to extend downward from the bottom surface of the cover that faces the upper surface of the chamber body.

Here, the detection well sealing portion is configured to be able to be forcibly fitted into the inlet of the detection well.

Here, the detection well sealing portion includes a detection well sealing member that is forcibly fitted into the inlet of the detection well to come into close contact with the inner surface of the detection well.

Here, the detection well sealing member is coupled to the body of the detection well sealing portion by using a double injection molding method.

Here, the cover has a light-transmitting portion capable of transmitting detection light into the detection well.

Here, the detection well sealing portion is provided to extend downward from the bottom surface of the cover that faces the upper surface of the chamber body, and the cover has a detection groove recessed on the upper surface in the protruding direction of the detection well sealing portion.

Here, the cover has a periphery sealing portion that is seated around the detection chamber while approaching the chamber body.

Here, the detection chamber includes an inclined portion that guides the sample to the detection well.

The inclined portion of the detection chamber has a micropattern that reduces the coefficient of surface friction.

Here, the inclined portion of the detection chamber has a guide protrusion that guides the sample to the center in the widthwise direction.

Here, at least one of the chamber body and the cover is provided to be rotatable relative to each other, and the inclined portion of the detection chamber is positioned closer to the center of the chamber body than the detection well.

Here, the detection chambers are arranged parallel to each other, such that a straight line having a certain angle to any one radial direction of the chamber body passes through the detection wells.

Here, the detection chamber includes an inclined portion that guides the sample to the detection well, and is provided in the form of an elongated hole extending in a direction parallel to the above radial direction, and the detection well is positioned farther away in the radial direction than the inclined portion.

iv) In order to achieve the above objectives, a fourth embodiment of the present disclosure may provide a nucleic acid detection cartridge, including a chamber body that is provided in a shape of a circular disk or a shape of a portion of a disk, and includes a plurality of chambers that are arranged in a circumferential direction with respect to a central axis, and include a sample accommodation chamber, and detection chambers in which nucleic acids are detected, wherein the detection chambers are arranged side by side such that a straight line having a certain angle to any one radial direction of the chamber body passes through the detection chambers.

Here, the nucleic acid detection cartridge further includes a pipette tip coupled to an end portion of a pipette that provides positive pressure and negative pressure, and the pipette tip withdraws the sample from the sample accommodation chamber and injects the sample into the detection chamber.

Here, the detection chambers are arranged such that the pipette performing a relative circular motion at a certain radius away from a central axis of the chamber body passes through the detection chambers.

Here, the detection chamber includes a detection well in which a nucleic acid is detected, and an inclined portion that guides a sample to the detection well.

Here, the inclined portion is provided to be inclined downward toward the detection well, and the detection well is provided deeper than the inclined portion.

Here, the inclined portion includes a micropattern that reduces the coefficient of surface friction.

Here, the inclined portion includes a guide protrusion that guides the sample to the center in the widthwise direction.

Here, the nucleic acid detection cartridge further includes a cover provided to cover the upper surface of the chamber body, and at least one of the chamber body and the cover is provided to be rotatable relative to each other about an axis of rotation.

Here, the inclined portion is positioned closer to the center of the chamber body than the detection well.

Here, the detection chamber is provided in a form of an elongated hole extending in a direction parallel to the above radial direction, and the detection well is positioned farther away in the radial direction than the inclined portion.

The cover has a detection well sealing portion capable of sealing the detection well.

Here, at least one of the chamber body and the cover is able to be selectively positioned relative to each other in a first position where the detection well sealing portion opens the detection well, and a second position where the detection well sealing portion seals the detection well.

Here, at least one of the chamber body and the cover is provided to be able to translate relative to each other in a direction of the axis of rotation.

Here, the detection well sealing portion is provided as a plug inserted into an inlet of the detection well.

Here, the detection well sealing portion is provided to extend downward from the bottom surface of the cover that faces the upper surface of the chamber body.

Here, the detection well sealing portion is configured to be able to be forcibly fitted into the inlet of the detection well.

Here, the detection well sealing portion includes a detection well sealing member that is forcibly fitted into the inlet of the detection well to come into close contact with the inner surface of the detection well.

Here, the detection well sealing portion is provided in a shape that protrudes toward the bottom of the cover, and the cover has a detection groove that is recessed from the upper surface in the protruding direction of the detection well sealing portion.

Here, the cover has a light-transmitting portion capable of transmitting detection light into the detection well.

Here, the cover has a periphery sealing portion that is seated around the detection chamber while approaching the chamber body.

In addition, there may be provided a nucleic acid detection cartridge, including a chamber body that is provided in a shape of a circular disk or a shape of a portion of a disk, and includes a plurality of chambers that are arranged in a circumferential direction with respect to a central axis, and include a sample accommodation chamber, and detection chambers in which nucleic acids are detected, wherein the detection chamber includes a detection well where a nucleic acid is detected, and an inclined portion that guides a sample to the detection well, the inclined portion is provided to be inclined downward toward the detection well, and the detection well is provided deeper than the inclined portion.

Here, the inclined portion has a micropattern that reduces the coefficient of surface friction.

Here, the inclined portion includes a guide protrusion that guides the sample to the center in the widthwise direction.

Here, the nucleic acid detection cartridge further includes a cover provided to cover the upper surface of the chamber body, and at least one of the chamber body and the cover is provided to be rotatable relative to each other about an axis of rotation.

Here, the inclined portion is positioned closer to the center of the chamber body than the detection well.

Here, the detection chambers are arranged parallel to each other, such that a straight line having a certain angle to any one radial direction of the chamber body passes through the detection wells.

Here, the detection chamber is provided in a form of an elongated hole extending in a direction parallel to the radial direction, and the detection well is positioned farther away in the radial direction than the inclined portion.

Here, the cover has a detection well sealing portion capable of sealing the detection well, and at least one of the chamber body and the cover is able to be selectively positioned relative to each other in a first position where the detection well sealing portion opens the detection well, and a second position where the detection well sealing portion seals the detection well.

Here, at least one of the chamber body and the cover is provided to be able to translate relative to each other in a direction having a certain angle to the upper surface.

Here, at least one of the chamber body and the cover is provided to be movable relative to each other on a plane parallel to the upper surface.

Here, at least one of the chamber body and the cover is provided to be rotationally movable relative to each other, and at least one of the chamber body and the cover is provided to be able to translate relative to each other in a direction of the axis of rotation.

Here, the detection well sealing portion is provided as a plug capable of sealing an inlet of the detection well.

Here, the detection well sealing portion is provided to seal the detection well as any one of the chamber body and the cover comes into proximity to each other in the direction of the axis of rotation.

Here, the detection well sealing portion is provided to extend downward from the bottom surface of the cover that faces the upper surface of the chamber body.

Here, the detection well sealing portion is provided to be forcibly fitted into the inlet of the detection well.

Here, the detection well sealing portion includes a detection well sealing member that is forcibly fitted into the inlet of the detection well to come into close contact with the inner surface of the detection well.

Here, the detection well sealing member is coupled to the detection well sealing portion by using a double injection molding method.

Here, the detection well sealing portion is provided to extend downward from the bottom surface of the cover that faces the upper surface of the chamber body, and the cover has a detection groove recessed on the upper surface in the protruding direction of the detection well sealing portion.

Here, the cover has a light-transmitting portion capable of transmitting detection light into the detection well.

Here, the cover has a periphery sealing portion that is seated around the detection chamber while approaching the chamber body.

### ADVANTAGEOUS EFFECTS

i) According to an embodiment of the present disclosure, a cover may facilitate aspiration and withdrawal of samples or reagents while preventing leakage in chambers.

In addition, the relative positions of a chamber body and the cover may be changed in a direction on a plane as well as in a direction perpendicular to the plane, thereby preventing gas leakage during a detection process.

According to another embodiment of the present disclosure, the cover may facilitate aspiration and withdrawal of a sample or a reagent through a pipette tip that moves together with the cover while preventing leakage in the chambers by using the cover.

In addition, by allowing the pipette tip to vertically move relative to the cover, the pipette tip may be prevented from being broken even when the cover and the chamber body move relative to each other.

ii) According to another embodiment of the present disclosure, crude samples that have not undergone any sample purification process may be used as is, and a self-filtering system may be provided to obtain a highly accurate target analyte detection result.

In addition, a sample accommodation chamber and a sample withdrawal chamber may be separated and equipped with a filter to obtain a refined sample.

iii) According to another embodiment of the present disclosure, a cover may facilitate aspiration and withdrawal of samples or reagents while preventing leakage in the chambers.

In addition, a detection well plug provided in the cover may be changed in relative position with respect to a detection well, thereby preventing gas leakage during a detection process.

iv) According to another embodiment of the present disclosure, unlike a plurality of chambers arranged in a circumferential direction, a plurality of detection chambers are arranged on a straight line, thereby improving the detection rate and accuracy in a structure in which a detection module moves in advance.

In addition, the detection module may perform detection while moving in advance in a state in which the chamber body is stationary, and thus, the detection rate and accuracy may be improved.

According to another embodiment of the present disclosure, a region where a sample is injected by a pipette in a detection chamber, and a region where detection is performed may be spatially separated from each other.

In addition, as the detection chamber includes an inclined surface, a sample injected into the detection chamber may flow into the detection well in total.

In addition, by providing the detection chamber with a double structure, an exact amount of sample may be distributed to all of a plurality of detection chambers in a structure in which the chamber body rotates circularly and the detection module moves in advance.

The effects of the present disclosure are not limited to the foregoing, and should be understood to include all effects that may be inferred from the detailed description of the present disclosure or the configuration described in claims.

### BRIEF DESCRIPTION OF THE DRAWINGS

FIG. 1 is a perspective view showing a nucleic acid detection cartridge according to an embodiment.
FIG. 2 is an exploded perspective view of FIG. 1.
FIG. 3 is a diagram showing a view of FIG. 2 from below.
FIG. 4 is a perspective view showing a chamber body equipped with a sealing film.
FIG. 5 is a perspective view of a chamber body.
FIG. 6 is a plan view of a chamber body.
FIG. 7 is a plan view showing a cover transparently at a sample injection position.
FIG. 8 is a plan view showing a cover transparently at a sample detection position.
FIG. 9 is a cross-sectional view at a sample detection position.
FIG. 10 is a perspective view showing a sample accommodation chamber.
FIG. 11 is a cross-sectional view showing a sample accommodation chamber at a sample injection position.
FIG. 12 is a cross-sectional view showing a sample accommodation chamber at a sample withdrawal position.
FIG. 13 is a plan view showing a bead well.
FIG. 14 is a cross-sectional view showing a bead well.
FIG. 15 is a plan view showing a detection chamber.
FIG. 16 is a cross-sectional view of a detection line in FIG. 15.
FIG. 17 is a cross-sectional view showing a detection chamber.
FIG. 18 is a side view showing a rotation prevention structure.
FIG. 19 is a cross-sectional view showing a separation prevention structure.
FIG. 20 is a cross-sectional view showing a sealing ring.

### DETAILED DESCRIPTION

Hereinafter, the present disclosure will be described in detail with reference to embodiments and example drawings. The embodiments are for illustrative purposes only, and it should be apparent to those of skill in the art that the scope of the present disclosure is not limited to the embodiments.

In addition, in adding reference numerals to the components of each drawing, it should be noted that same reference numerals are assigned to same components as much as possible even though they are shown in different drawings. In addition, in describing the embodiments of the present disclosure, when it is determined that a detailed description of a related well-known configuration or function interferences with the understanding of the embodiments of the present disclosure, the detailed description thereof will be omitted.

In addition, in describing the components of the embodiments of the present disclosure, terms such as first, second, A, B, (a), (b), (i), (ii), etc. may be used. These terms are only for distinguishing the components from other components, and the nature or order of the components is not limited by the terms. When a component is described as being "connected," "coupled" or "fastened" to other component, the component may be directly connected or fastened to the other component, but it will be understood that another component may be "connected," "coupled" or "fastened" between the components.

The present disclosure relates to a detection device for detecting a target analyte in a sample.

As used herein, the term "sample" may include a biological sample (e.g., cells, tissues, and fluids from a biological source) and a non-biological sample (e.g., food, water and soil). Examples of the biological sample may include viruses, bacteria, tissues, cells, blood (e.g., whole blood, plasma, and serum), lymph, bone marrow fluid, saliva, sputum, swab, aspiration fluid, milk, urine, feces, ocular fluid, semen, brain extract, spinal fluid, joint fluid, thymic fluid, bronchoalveolar lavage fluid, ascites, and amniotic fluid. In addition, the sample may include natural nucleic acid molecules isolated from a biological source, and synthetic nucleic acid molecules. In the present specification, the sample may include an additional substance such as water, deionized water, saline solution, pH buffer, acid solution, or alkaline solution.

A target analyte refers to a substance that is the subject of analysis. The analysis may refer to obtaining information about, for example, the presence or absence, content, concentration, sequence, activity, or property of the analyte in the sample. The analyte may include various substances (e.g., biological substances and non-biological substances such as compounds). In detail, the analyte may include a biological substance such as nucleic acid molecules (e.g., deoxyribonucleic acid (DNA) and ribonucleic acid (RNA)), proteins, peptides, carbohydrates, lipids, amino acids, biological compounds, hormones, antibodies, antigens, metabolites, or cells. In the present specification, the analyte may be nucleic acid molecules.

In addition, in the present specification, the sample may include an optical label. The optical label refers to a label that generates an optical signal depending on the presence of a target nucleic acid. The optical label may be a fluorescent label. In the present specification, the fluorescent label may include any molecule known in the art.

Thus, a target analyte detection device of the present specification may be a target nucleic acid detection device. The target nucleic acid detection device causes a nucleic acid reaction to occur in a sample, to detect a target nucleic acid.

The nucleic acid reaction refers to a series of physical and chemical reactions that generate a signal depending on the presence or amount of a nucleic acid of a specific sequence in the sample. The nucleic acid reaction may include the binding of a nucleic acid of a specific sequence in a sample to other nucleic acids or substances, and replication, cleavage or decomposition of a nucleic acid of a specific sequence in the sample. The nucleic acid reaction may involve a nucleic acid amplification reaction. The nucleic acid amplification reaction may include amplification of a target nucleic acid. The nucleic acid amplification reaction may specifically amplify the target nucleic acid.

The nucleic acid reaction may be a signal-generation reaction that may generate a signal depending on the presence/absence or amount of a target nucleic acid in a sample. The signal-generation reaction may be a process of genetic analysis such as polymerase chain reaction (PCR), real-time PCR, or microarray.

Various methods of generating an optical signal, which indicates the presence of a target nucleic acid, by using a nucleic acid reaction are known. Representative examples thereof include the following: TaqMan^{™} probe method (U.S. Pat. No. 5,210,015), molecular beacon method (Tyagi et al., Nature Biotechnology v.14 March 1996), Scorpion method (Whitcombe et al., Nature Biotechnology 17:804-807(1999)), Sunrise or Amplifluor method (Nazarenko et al., 2516-2521 Nucleic Acids Research, 25(12):2516-2521(1997), and U.S. Pat. No. 6,117,635), Lux method (U.S. Pat. No. 7,537,886), CPT (Duck P, et al., Biotechniques, 9:142-148(1990)), LNA method (U.S. Pat. No. 6,977,295), Plexor method (Sherrill CB, et al,, Journal of the American Chemical Society, 126:4550-4556(2004)), Hybeacons^{™} (D. J. French, et al., Molecular and Cellular Probes (2001) 13, 363-374 and U.S. Pat. No. 7,348,141), dual-labeled, self-quenched probe (U.S. Pat. No. 5,876,930), hybridization probe (Bernard PS, et al., Clin Chem 2000, 46, 147-148), PTOCE (PTO cleavage and extension) method (WO 2012/096523), PCE-SH (PTO Cleavage and Extension-Dependent Signaling Oligonucleotide Hybridization) method (WO 2013/115442), PCE-NH (PTO Cleavage and Extension-Dependent Non-Hybridization) method (PCT/KR2013/012312), and CER method (WO 2011/037306).

A target analyte detection device according to an embodiment of the present specification may be a nucleic acid detection device, and may detect a signal that is generated depending on the presence of a target nucleic acid. The nucleic acid detection device may amplify and detect a signal with nucleic acid amplification. Alternatively, the nucleic acid detection device may amplify and detect a signal without nucleic acid amplification. Preferably, the nucleic acid detection device detects a signal with nucleic acid amplification.

A target analyte detection device according to an embodiment of the present specification may include a nucleic acid amplification device.

The nucleic acid amplification device refers to a device capable of performing a nucleic acid amplification reaction to amplify a nucleic acid having a specific nucleotide sequence. Examples of methods of amplifying a nucleic acid include PCR, ligase chain reaction (LCR) (U.S. Pat. Nos. 4,683,195 and 4,683,202; PCR Protocols: A Guide to Methods and Applications (Innis et al., eds, 1990)), strand displacement amplification (SDA) (Walker, et al. Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatch et al., Genet. Anal. 15(2):35-40 (1999)), Q-beta Replicase (Lizardi et al., BiolTechnology 6:1197 (1988)), etc.

A target analyte detection device according to an embodiment may be a device for performing a nucleic acid amplification reaction while causing a change in temperature. For example, the nucleic acid amplification device may perform a denaturing step, an annealing step, and an extension (or amplification) step, to amplify DNA having a specific base sequence.

In the denaturing step, a solution containing a reagent and a sample containing double-stranded DNA, which is a template nucleic acid, is heated to a specific temperature, for example, about 95 °C, to separate the double-stranded DNA into single-stranded DNA. In the annealing step, an oligonucleotide primer having a nucleotide sequence complementary to the nucleotide sequence of a nucleic acid to be amplified is provided, and the primer and the separated single-stranded DNA are cooled down to a specific temperature, for example 60 °C, to bind the primer to a specific nucleotide sequence of the single-stranded DNA to form a partial DNA-primer complex. In the extension step, the solution is maintained at a specific temperature, for example 72 °C, after the annealing step, to form double-stranded DNA by DNA polymerase based on the primer of the partial DNA-primer complex.

The above-described three steps are repeated, for example, 10 to 50 times, to exponentially amplify DNA having the specific nucleotide sequence. In some cases, the nucleic acid amplification device may perform the annealing step and the extension step simultaneously. In this case, the nucleic acid amplification device may complete one cycle by performing two steps including a denaturing step and an annealing/extension step.

In particular, a target analyte detection device according to an embodiment may be a device for performing a nucleic acid amplification reaction and a reaction that generates an optical signal depending on the presence of a nucleic acid, while causing a change in temperature, and detecting the generated optical signal.

In addition, a target analyte detection device according to an embodiment may be a point-of-care (POC) diagnostic device, which is a miniaturized on-site diagnostic device.

The target analyte detection device may include a holding module for holding a cartridge, a fluid transportation module, an extraction module, a thermo-control module, a sensing module, and a controller for controlling these modules. The holding module may include a fixed type that allows a user to manually hold a cartridge at a predetermined position, and a moving type that receives a cartridge from the outside of the detection device and automatically holds the cartridge at a predetermined position.

The fluid transportation module may provide power for transporting a fluid including a sample, a reagent, or a buffer. The term 'reagent' may have a meaning distinct from that of the term 'buffer', and in some cases, may be used to encompass the concept of 'buffer'.

In a case in which a cartridge is provided in a well type, fluid transportation may be performed by using a syringe pump. Alternatively, in a case in which a cartridge is provided in a micro-fluidic type, fluid transportation may be performed by using a pump and a valve inside or outside a microdevice. Here, a micropump and microvalves often require an additional driving force. Examples of driving mechanisms for a micropump include check valves or peristaltic, rotary, centrifugal, ultrasonic, electro-hydrodynamic, electro-kinetic, phase transfer (requiring a change in temperature or pressure), electrowetting, magnetic, or hydrodynamic mechanisms, etc. Examples of driving mechanisms for microvalves include pneumatic, thermopneumatic, thermomechanical, piezoelectric, electrostatic, electromagnetic, electrochemical, or capillary mechanisms, and the like (Reference examples: U.S. Pat. Nos. 6,531,417; 5,499,909; Kamper, K. P. et al., "A self-filling low-cost membrane micropump", The 11th annual international workshop on MEMS, 1998 Heidelberg Germany, 432-437; Maillefer, D. et al., "A high-performance silicon micropump for disposable drug delivery systems", The thirteenth IEEE International Micro Electro Mechanical Systems (MEMS) 2000 Conference, Miyazaki, Japan, 413-417; Gu, W. et al., Proc. Natl. Acad. Sci. U.S.A (2004), 101, 45, 15861-15866).

The extraction module may be used to extract a target analyte from a sample. For example, the extraction module may be used to extract a nucleic acid from a sample. In addition, the extraction module may include a magnet used to manipulate magnetic beads to which a nucleic acid is bound, and a mixing means used to mix a sample with a reagent in an extraction process. For example, the mixing means may include an ultrasonic horn.

The thermo-control module may be used to control the temperature in a process such as a target analyte extraction reaction or a target analyte detection reaction. For example, the thermo-control module may be used to control the temperature in a nucleic acid extraction reaction or a nucleic acid amplification reaction. In addition, the thermo-control module may include a heating element, a heat sink, a cooling fan, or a temperature sensor. For example, the heating element may include a Peltier element or a heating wire.

The sensing module may be used to sense a target analyte. Sensing of a target analyte may be performed by using an optical method or a chemical method. For example, the sensing module may be an optical module or an electrochemical module.

The optical module may detect a target analyte by using such a method as fluorescence or color change sensing, or absorbance or reflectance measurement. For example, the optical module may detect fluorescence emitted from a fluorescent material labeled on a target nucleic acid sequence.

In addition, the optical module may include a light-emitting unit for supplying an appropriate optical stimulus to a sample accommodated in a sample holder, and a detection unit for detecting an optical signal generated from the sample in response to the optical stimulus.

The optical signal may be luminescence, phosphorescence, chemiluminescence, fluorescence, polarized fluorescence, or colored signal. The optical signal may occur in response to the optical stimulus applied to the sample. For example, the light-emitting unit may include a light-emitting diode (LED) or a laser, and the detection unit may include a charge-coupled device (CCD), a complementary metal-oxide-semiconductor (CMOS) field-effect transistor, a photodiode, etc.

The electrochemical module may electrically sense the occurrence of a chemical reaction or a change in the degree of a chemical reaction according to the presence or absence of a target analyte in a sample, or a change in the amount of the target analyte. For example, the electrochemical module may sense a change in the target analyte in the sample by sensing a change in pH or resistance according to an increase in the target analyte, or by sensing an electrochemical signal generated by binding of the target analyte and an active material. The electrochemical module may include an electrode unit and an electrical signal measurement unit.

A detection electrode may be made of, for example, gold (Au), cobalt (Co), platinum (Pt), silver (Ag), carbon nanotubes, graphene, carbon (C), or an alloy including one or more thereof.

The electrical signal measurement unit may include, for example, anodic stripping voltammetry (ASV), chronoamperometry (CA), cyclic voltammetry, square wave voltammetry (SWV), differential pulse voltammetry (DPV) or impedancemetry.

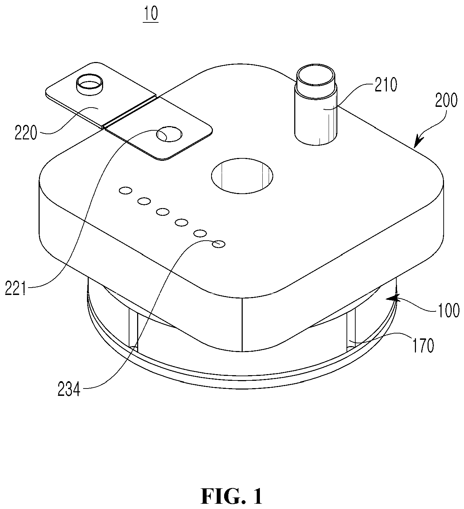

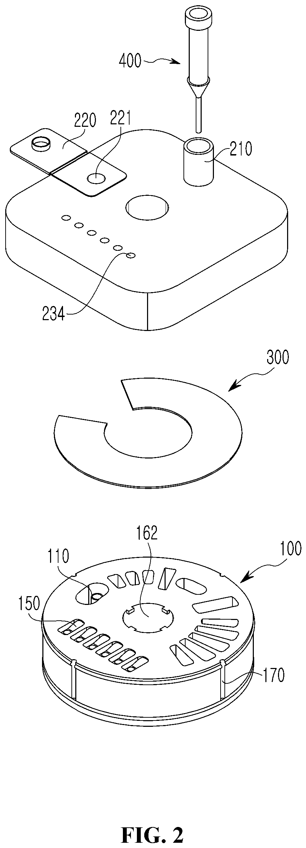

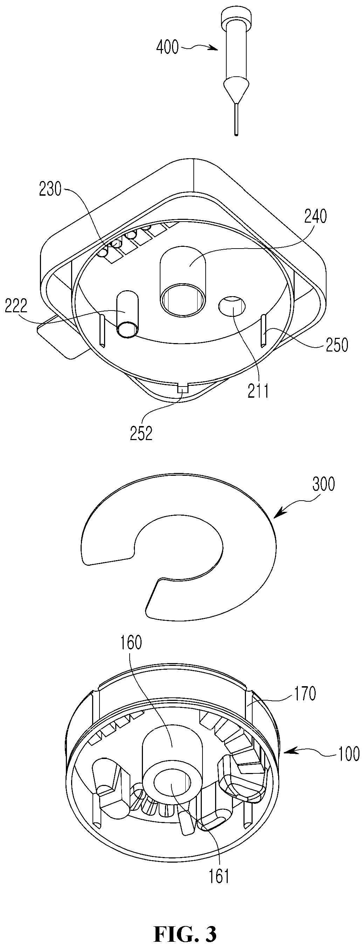

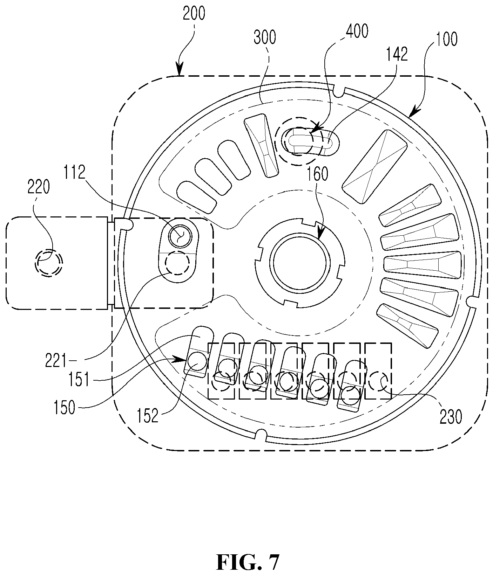

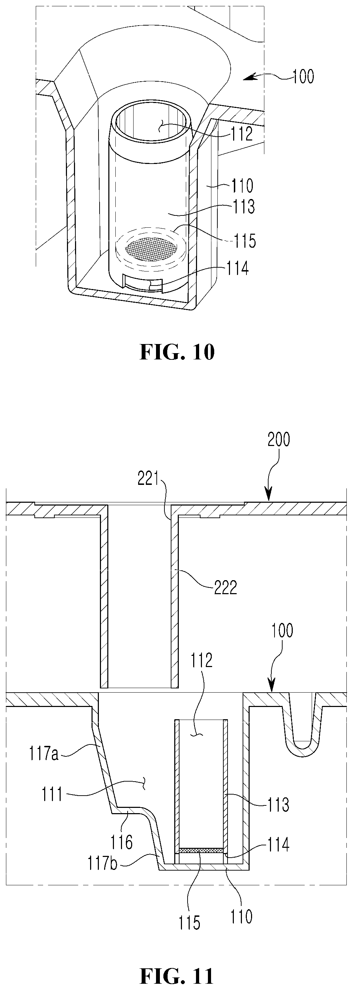

A cartridge 10 according to an embodiment of the present disclosure will be described with reference to FIGS. 1 to 18.

The cartridge 10 according to an embodiment of the present disclosure may include a chamber body 100 capable of accommodating a sample, a reagent, etc., and a cover 200 that covers an upper portion of the chamber body 100. In addition, the cartridge 10 may further include a pipette tip 400 to be coupled to an end portion of a pipette capable of aspirating and injecting a fluid accommodated in the chamber body 100, such as a sample or a reagent.

The chamber body 100 may be provided with a plurality of chambers. The chambers may have an opening formed on an upper surface of the chamber body 100, and a fluid accommodation portion formed on a lower portion thereof.

The cover 200 may be provided to face all or part of the upper surface of the chamber body 100. In addition, the cover 200 may be provided to cover all or part of an outer rear surface of the chamber body 100. In an embodiment, the chamber body 100 may be provided in the shape of a circular disk or the shape of a portion of a disk, and the cover 200 may include an upper portion facing the upper surface of the chamber body 100, and an inner side surface facing an outer side surface of the chamber body 100. For example, in a case in which the chamber body 100 is provided in a cylindrical shape having an upper surface and an outer side surface, the cover 200 may include a cover surface that covers the upper surface of the chamber body 100, and a side surface that surrounds an outer side surface of the chamber body 100.

In addition, the cover 200 may be provided to cover the chambers of the chamber body 100. **In** addition, the cover 200 may be provided to seal the upper portions of the chambers of the chamber body 100. In addition, the cover 200 may prevent a material accommodated in the chamber from being separated out of the cartridge 10, and prevent foreign substances outside the cartridge 10 from entering the chamber. Furthermore, the cover 200 may also prevent a fluid or gas inside the chambers from escaping to the outside. In particular, while a reaction occurs in a detection well 152 to be described below, the cover 200 may seal the detection well 152 to prevent external contaminants from entering the detection well 152, to prevent a reactant inside the detection well 152 from leaking out, and furthermore, to prevent gas inside the detection well 152 from leaking out. To this end, a nucleic acid detection device may be equipped with a separate pressurizing unit (not shown) for pressurizing the cover 200 or the chamber body 100.

Meanwhile, at least one of the chamber body 100 and the cover 200 may be provided to be movable relative to each other in a direction on a plane parallel to one surface. Here, the one surface includes the upper surface of the chamber body 100 and a surface parallel to the upper surface. In addition, the movement in a direction on a plane includes rotational translation about an axis of rotation.

In addition, at least one of the chamber body 100 and the cover 200 may be provided to be movable relative to each other in one direction at a certain angle to one surface. Here, the one surface includes the upper surface of the chamber body 100 and a surface parallel to the upper surface. In addition, the one direction at a certain angle on one surface includes a direction perpendicular to the upper surface of the chamber body 100. In addition, the movement in one direction includes a vertical movement and a translation. In addition, the translation includes a translation in the direction of the axis of rotation.

In the present embodiment, the cover 200 may be provided to be movable up and down relative to the chamber body 100. That the cover 200 is movable up and down relative to the chamber body 100 means that the cover 200 is movable and/or the chamber body 100 is movable. In addition, the up-and-down movement includes not only a vertical movement, but also a movement in a direction at a certain angle to a vertical direction. In addition, the up-and-down movement may include moving to a position of proximity and moving to a position of contact. The up-and-down movement may be a translation, and may be in the direction of the axis of rotation. At least one of the cover 200 and the chamber body 100 may be provided to be able to translate relative to each other in the direction of the axis of rotation.

For example, the cover 200 may move downward. When the cover 200 moves downward such that the cover surface of the cover 200 comes into contact with the upper surface of the chamber body 100, or such that a portion protruding downward from the cover surface of the cover 200 comes into contact with the chambers of the chamber body 100 or the periphery of the chamber body 100, the cover 200 may seal one or more of the chambers.

In the present embodiment, the chamber body 100 may be provided to be rotationally movable relative to the cover 200. That the cover 200 is rotationally movable relative to the chamber body 100 means that the cover 200 is rotatable and/or the chamber body 100 is rotatable.

For example, the cover 200 may be provided to be fixed, and the chamber body 100 may be provided to rotate.

In more detail, the chamber body 100 may be provided to be rotatable about an axis of rotation, and the cover 200 may be provided to surround the outer side surface of the chamber body 100 while allowing rotation of the chamber body 100. For example, the chamber body 100 may be provided in a circular shape when viewed from above, and the cover 200 may include an inner side surface provided to surround the outer side surface of the chamber body 100. For example, the chamber body 100 may be provided in the shape of a circular disk or the shape of a portion of a disk. The shape of a circular disk is a shape that is circular when viewed from above and has a volume, and the shape of a portion of a disk is a fan shape that is surrounded by two radii and one arc when viewed from above, and has a volume. The central angle formed by the two radii is not particularly limited, but may be, for example, 45 degrees, 60 degrees, 90 degrees, or 180 degrees.

The chamber body 100 may be rotatable in one direction or in both directions with respect to the central axis. For example, the chamber body 100 may be arranged to be able to rotate about 360 degrees in one direction, or may be arranged to be able to rotate about 180 degrees clockwise and about 180 degrees counterclockwise. In addition, the angle and direction of rotation may be variously provided depending on the design of the chamber body 100.

The rotational driving force of the chamber body 100 may be transmitted through a lower portion of the chamber body 100. To this end, the nucleic acid detection device may be equipped with a separate rotation driving unit (not shown) that rotates the chamber body 100. For example, the chamber body 100 may be coupled to a drive shaft of the rotation driving unit, at a central portion thereof.

According to a basic embodiment to be described below, the chamber body 100 may be provided to be rotatable, and the cover 200 may be provided to be vertically movable. However, instead, the cover 200 may be provided to be rotatable, and the chamber body 100 may be provided to be vertically movable. Alternatively, various embodiments are possible, for example, the chamber body 100 or the cover 200 may be provided to be rotationally movable and vertically movable.

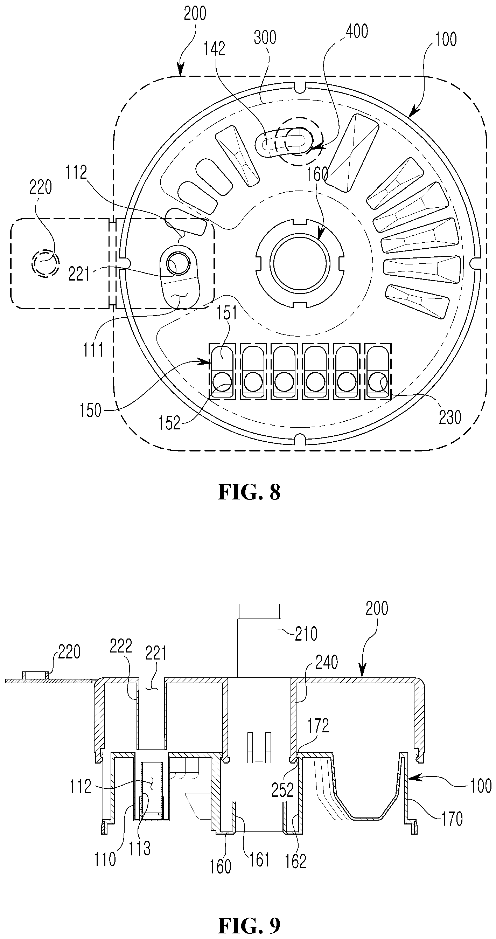

Referring to FIG. 9, the chamber body 100 may include a mounting portion 160 into which the drive shaft of the rotation driving unit (not shown) may be inserted, around the center of the axis of rotation. The mounting portion 160 may include a collar portion 161 extending upward along the central axis from the lower surface of the chamber body 100. Alternatively, the collar portion 161 may extend downward along the central axis from the upper surface of the chamber body 100.

In addition, the mounting portion 160 may include a connecting groove 162 that accommodates a connecting rod 240 extending downward around the central axis of the cover 200. For example, the connecting groove 162 may be provided on the outside of the collar portion 161. The connecting rod 240 may be guided into the connecting groove 162 to vertically move inside the connecting groove 162.

Alternatively, conversely, a connecting groove may be formed in the cover 200 and a connecting rod may be provided in the chamber body 100.

Meanwhile, the nucleic acid detection device is equipped with a pipette (not shown) that provides aspiration pressure and injection pressure. For example, the pipette may be connected to a pump (not shown) that provides pneumatic pressure.

The cartridge 10 may include the pipette tip 400 to be coupled to an end portion of the pipette. The pipette tip 400 may move integrally with the pipette, and may transmit the pneumatic pressure of the pipette to a fluid accommodated in the chamber of the chamber body 100.

In addition, the pipette tip 400 may be coupled to be vertically movable with respect to the cover 200. To this end, the cover 200 may form a pipette hole 211 through which the pipette or the pipette tip 400 may enter and exit.

At least one of the chamber body 100 and the cover 200 may be provided to be rotatable relative to each other, and accordingly, the pipette tip 400 may change its relative position in the circumferential direction of the chamber body 100 together with the cover 200 to transfer fluids between the plurality of chambers. In a state in which the lower end of the pipette tip 400 is positioned above the upper surface of the chamber body 100, the chamber body 100 may be allowed to rotationally move. A controller for controlling rotational driving of the nucleic acid detection cartridge 10 may align the position of the chamber body 100 such that the pipette tip 400 is positioned above a target chamber. Thus, the pipette may withdraw fluids from all chambers of the chamber body 100 or inject fluids into all chambers of the chamber body 100, through the pipette tip 400 only by vertical movement without moving in a direction on a plane, including revolution.

The chamber body 100 may include a plurality of chambers arranged in the circumferential direction with respect to the axis of rotation. Some of the plurality of chambers may have a shape extending in the radial direction of the chamber body 100. For example, some of the plurality of chambers may be provided in the shape of an elongated hole with a radial length greater than a circumferential width.

In addition, some of the plurality of chambers may have a shape whose cross-sectional area narrows toward the bottom. For example, some of the plurality of chambers may have a shape whose circumferential width narrows toward the bottom. Alternatively, some of the plurality of chambers may have a shape whose radial width narrows toward the bottom.

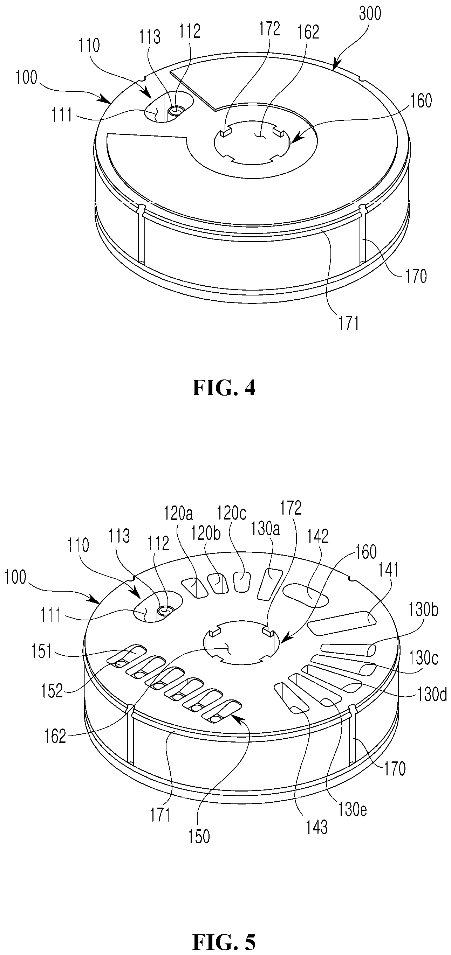

In addition, referring to FIG. 4, the cartridge 10 according to an embodiment of the present disclosure may further include a sealing film 300 that covers openings of the plurality of chambers exposed from the upper portion of the chamber body 100. The sealing film 300 may be provided to seal inlets of the chambers after inserting reagents, buffers, or beads into the chambers.

The sealing film 300 may be a thin membrane, for example, an aluminum (Al) foil. In addition, the sealing film 300 may be attached to the upper surface of the chamber body 100 by performing thermal fusion or by using an adhesive or the like.

The sealing film 300 may be configured to be penetrated by the pipette tip. In other words, the sealing film 300 is provided to be broken by a tip portion of the pipette tip 400. Alternatively, in a case in which a chamber sealing portion (not shown) protruding from the lower surface of the cover 200 is provided, the sealing film 300 may be broken by the pressure applied by the chamber sealing portion as the cover 200 descends. The chamber sealing portion may descend while tearing the sealing film 300 to be accommodated in the chambers. In some cases, the sealing film 300 may have dot- or line-shaped grooves provided in advance in portions thereof to be cut, to facilitate breakage of the sealing film 300.

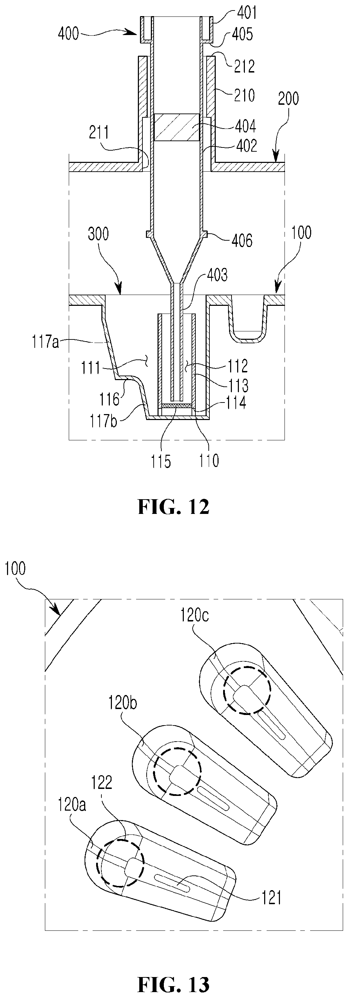

Referring to FIG. 12, the pipette tip 400 may be coupled to the cover 200 to be vertically movable. In addition, a pipette may be coupled to an upper portion of the pipette tip 400, and the pipette tip 400 may transmit negative or positive pressure provided by the pipette.

In addition, the tip portion of the pipette tip 400 may be formed in the shape of a thin tube. The tip portion of the pipette tip 400 is able to easily penetrate the sealing film 300, and enables fine control of capacity.

The pipette tip 400 may include a connecting portion 401 to which the pipette is coupled, a pipette tip body 402 extending downward from the connecting portion 401 to form a flow path, a tip portion 403 extending downward from the pipette tip body 402, and a pipette tip filter member 404 provided in the middle of the flow path inside the pipette tip body 402.

The cover 200 may further include a pipette tip guide 210 that guides a vertical movement of the pipette tip 400. The pipette tip guide 210 may extend along a movement path of the pipette tip 400. For example, the pipette tip guide 210 may be arranged to protrude upward from the upper surface of the cover 200, and may accommodate therein the pipette tip 400. Because the cover 200 comes into proximity to or into close contact with the chamber body 100 when detecting a nucleic acid, there may be insufficient space below the upper surface of the cover 200 for the pipette tip guide 210 to extend.

In addition, the cover 200 may further include the pipette hole 211 provided on the movement path of the pipette or the pipette tip 400 to aspirate fluids from one or more of the plurality of chambers and inject fluids into one or more of the plurality of chambers. The pipette hole 211 formed in the cover 200 may be provided inside the pipette tip guide 210, and for example, the pipette hole 211 may be formed to penetrate the upper surface of the cover 200. The pipette tip 400 may enter and exit through the pipette hole 211. The cover 200 and the chamber body 100 move relative to each other, and the pipette tip 400 transfers solutions accommodated in the chambers included in the chamber body 100. Thus, from the perspective of the chamber body 100, the pipette hole 211 may be provided on the movement path of the pipette or the pipette tip 400.

In addition, the pipette tip 400 is coupled to an end portion of the pipette and is provided to be able to move vertically through the pipette hole 211 of the cover. Thus, the diameter of the pipette hole 211 may be greater than the diameter of a horizontal cross-section of the pipette tip body 402. The pipette tip 400 may be provided such that its outer side surface has the shape of a cylinder or a portion of a cylinder, and the pipette tip guide 210 may be provided such that its inner side surface has the shape of a cylinder or a portion of a cylinder, which corresponds to the outer side surface of the pipette tip 400. In addition, the inner diameter of the pipette tip guide 210 may be set slightly larger than the outer diameter of the pipette tip 400 so as to reduce friction between the pipette tip guide 210 and the pipette tip 400.

In addition, the pipette tip guide 210 may be provided with a support jaw 212 that supports the pipette tip 400 to prevent the pipette tip 400 from being separated downward, and the pipette tip 400 may be provided with a first catching protrusion 405 that protrudes outward to be supported by the support jaw 212.

For example, the first catching protrusion 405 may have the shape of a flange that protrudes outward from the outer side surface of the pipette tip 400. In addition, in a state in which the cover 200 is spaced from the chamber body 100, when the first catching protrusion 405 of the pipette tip 400 is supported by the pipette tip guide 210, the tip portion of the pipette tip 400 may be spaced from the bottom of the chambers. Thus, when the pipette tip 400 moves downward, the tip portion of the pipette tip may be prevented from colliding with the bottom of the chambers to be damaged.

In addition, the chambers of the chamber body 100 may have the same depth, and the tip portion 403 provided in a tubular shape may be provided to have a length that is greater than or equal to the depth of the chambers. Thus, it is possible to prevent the sealing film 300 from being damaged excessively when the tip portion 403 penetrates the sealing film 300 covering the upper portions of the chambers to enter.

The pipette tip 400 may further include therein the pipette tip filter member 404. The pipette tip filter member 404 may prevent external contaminants from entering the cartridge 10. The pipette tip filter member 404 may be positioned inside the pipette tip body 402, and a sufficient volume may be secured below the pipette tip filter member 404. Thus, when the pipette tip 400 aspirates a fluid from the chamber, the fluid may not come into contact with the pipette tip filter member 404. This is because, if the fluid aspirated through the tip portion 403 comes into contact with the pipette tip filter member 404, the fluid may be exposed to contaminants filtered out by the pipette tip filter member 404.

In addition, a second catching protrusion 406 may be provided on the outer side surface of the pipette tip 400 to prevent the pipette tip 400 from being separated upward. In detail, in order to prevent the pipette tip from being separated upward from the cover, the pipette tip body 402 may be provided with the second catching protrusion 406 extending toward the outside of the opening of the pipette tip guide 210. The second catching protrusion 406 of the pipette tip 400 may protrude in a ring shape along the outer side surface of the pipette tip 400. In detail, the second catching protrusion 406 may be formed to protrude in the circumferential direction of the pipette tip body 402, and may be provided to be elastically deformable.

In addition, the second catching protrusion 406 may seal the space between the pipette tip 400 and a sidewall of the pipette tip guide 210. The second catching protrusion 406 may be formed integrally with the pipette tip 400, or may be provided as a separate elastic material. In addition, the second catching protrusion 406 may be formed on the pipette tip 400 by using a double injection method.

In addition, the inner side surface of the pipette tip guide 210 may include a step structure. The step structure may be arranged so that a lower inner diameter is greater than an upper inner diameter. The upper inner diameter of the pipette tip guide 210 may be greater than the outer diameter of the body of the pipette tip 400 and less than the outer diameter of the second catching protrusion 406. In addition, the lower inner diameter of the pipette tip guide 210 may correspond to the outer diameter of the second catching protrusion 406 of the pipette tip 400.

In addition, the second catching protrusion 406 of the pipette tip 400 may be provided to be elastically deformable. Thus, the pipette tip 400 may be assembled through an upper portion of the pipette tip guide 210 in an interference fit manner, and the second catching protrusion 406 may be elastically restored after passing through the step structure of the pipette tip guide 210 to prevent reseparation.

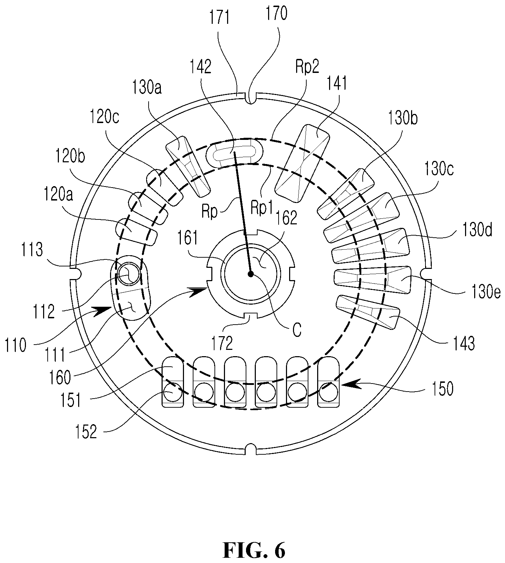

Referring to FIG. 6, the nucleic acid detection cartridge 10 may include a sample accommodation chamber 110 into which a sample is injected, a first bead chamber 120a in which proteinase K beads are accommodated, a second bead chamber 120b in which magnetic beads are accommodated, a third bead chamber 120c in which internal control beads are accommodated, a lysis buffer chamber 130a in which a lysis buffer is accommodated, a tip mounting chamber 142 that is a space in which a tip is mounted, a mixing chamber 141 in which fluids are mixed, a binding buffer chamber 130b in which a binding buffer is accommodated, a plurality of buffer chambers 130c, 130d, and 130e in which washing buffers are accommodated, and a dummy chamber 143 provided as a spare chamber. These chambers may be arranged in a circumferential direction (e.g., in the clockwise direction) in the above order.

In addition, the nucleic acid detection cartridge 10 may further include a plurality of detection chambers 150 in which nucleic acids included in linearly arranged samples are detected, in addition to the chambers arranged in the circumferential direction.

Meanwhile, when the distance between the pipette tip 400 and a center of rotation C is defined as a pipette radius Rp, the plurality of chambers may be provided such that the pipette radius Rp reaches the interiors of the plurality of chambers. For example, the plurality of chambers other than the detection chambers 150 may be arranged such that the center of each chamber is located at the pipette radius Rp. On the contrary, the plurality of detection chambers 150 may differ from each other in the distance from the center of rotation C to the center thereof. The center of rotation C may be the central axis of the chamber body 100. The detection chambers 150 may be arranged such that the pipette passes through the detection chambers 150 when performing a relative circular motion at a certain radius away from the central axis of the chamber body 100.

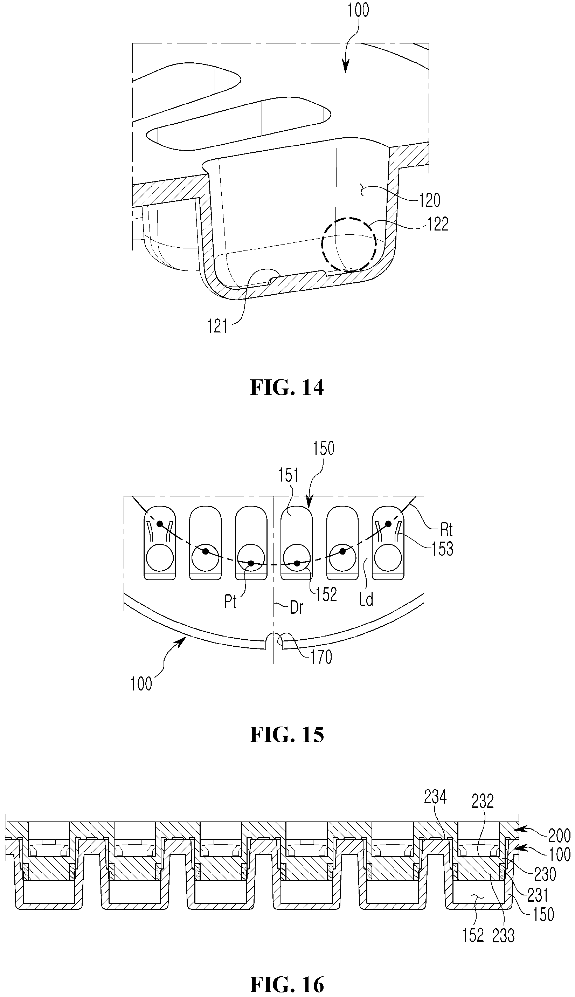

Referring to FIG. 15, the detection chambers 150 may be arranged such that a straight line Ld at a certain angle to a radial direction Dr of the chamber body 100 passes through the detection chambers 150. In addition, the plurality of detection chambers 150 may be arranged side by side at equal intervals in the direction of the straight line Ld.

This is to ensure that the plurality of detection chambers 150 are positioned on the progression path Ld of a detection module (not shown) of the nucleic acid detection device, since the detection module is provided to perform a linear motion.

For example, the distances between the center of rotation C and the centers of the respective detection chambers 150 may decrease toward both ends and increase toward the middle such that the distance between the center of rotation C and the center of the detection chamber 150 located in the center is the shortest, and the distance between the center of rotation C and the center of the detection chamber 150 located at each end is the longest.

Meanwhile, referring to FIG. 9, the cover 200 may have a sample injection hole 221 formed therein to enable sample injection into the sample accommodation chamber 110, and may include a stopper portion 220 that seals the sample injection hole 221. In addition, the stopper portion 220 may be integrally connected to the cover 200 so as to be prevented from being lost.

In addition, the cover 200 may further include a sample guide portion 222 extending toward the bottom of the sample injection hole 221. The sample guide portion 222 may block the surroundings such that a sample provided through the sample injection hole 221 may flow into the sample accommodation chamber 110. For example, the sample guide portion 222 may be provided in a cylindrical shape, and may have a shape and a width provided to allow a sample to be injected into the sample accommodation chamber 110. The sample injection hole 221 included in the cover 200 moves according to rotation of the cover 200, and may be formed to be aligned with the sample accommodation chamber 110 at a specific position. When the sample injection hole 221 is aligned with the sample accommodation chamber 110 formed in the chamber body 100, the sample accommodation chamber 110 may be opened.

Meanwhile, referring to FIG. 6, the sample accommodation chamber 110 may be provided in the form of an elongated hole extending in the circumferential direction. In the sample accommodation chamber 110, a position at which a sample is injected (hereinafter, referred to as a sample injection region 111) may be different from a position at which a sample is withdrawn (hereinafter, referred to as a sample withdrawal region 112). For example, in the sample accommodation chamber 110, the center of the sample injection region 111 and the center of the sample withdrawal region 112 may be arranged to be offset from each other by a certain angle, at the same radial distance from the center of rotation C. In addition, the center of the sample injection region 111 and the center of the sample withdrawal region 112 may be provided at the pipette radius Rp.

Referring to FIGS. 9 to 12, the sample accommodation chamber 110 may be spatially partitioned into the sample injection region 111 and the sample withdrawal region 112. By distinguishing between the region 111 where a sample is injected into the sample accommodation chamber 110, and the region 112 where the sample is withdrawn from the sample accommodation chamber 110, it is possible to primarily prevent floating substances or contaminants that may be included in the sample injected into the cartridge 10 from being withdrawn through the pipette.

In addition, the sample accommodation chamber 110 may be provided with a blocking portion that spatially separates the sample injection region 111 and the sample withdrawal region 112. For example, the blocking portion may be a blocking wall 113.

In addition, the sample accommodation chamber 110 may be provided with a sample flow channel 114 that penetrates the blocking wall 113. For example, the blocking wall 113 may be provided in a cylindrical shape, and the sample flow channel 114 may be a through hole penetrating a lower portion of the blocking wall 113. In addition, the sample flow channel 114 may be formed to penetrate both facing side surfaces of the blocking wall 113 to reduce sample flow resistance.

In addition, the height of the blocking wall 113 may be set to be higher than the height of a sample that completely fills the sample accommodation chamber 110, thereby restricting movement of the sample except for movement through the sample flow channel 114. In addition, the sample flow channel 114 may be provided close to the bottom of the sample accommodation chamber 110 so as to minimize the amount of sample that cannot move to the sample withdrawal region 112 and remains in the sample injection region 111.

That is, the sample inside the sample injection region 111 may flow into the sample withdrawal region 112 provided inside the blocking wall 113 only through the sample flow channel 114. In addition, the pipette may withdraw the sample from the sample withdrawal region 112. By providing the blocking wall 113 in the sample accommodation chamber 110 as described above, it is possible to secondarily prevent contaminants other than those that have settled to the bottom and are floating, from being withdrawn.

In addition, the sample accommodation chamber 110 may further include a filter member 115. The filter member 115 may be installed in the sample withdrawal region 112 or the sample flow channel 114. In addition, the pipette tip 400 for withdrawing a sample from the sample withdrawal region 112 may withdraw the sample that has been filtered while passing through the filter member 115 and then positioned above the filter member 115. In addition, the filter member 115 may be a sample filter 115 made of a porous material to filter a sample.

In detail, the sample injected into the sample injection region 111 of the sample accommodation chamber 110 may pass through the sample flow channel 114 and the sample filter 115 and then move to the sample withdrawal region 112. At this time, floating substances or contaminants in the sample may be finally removed while passing through the sample filter 115. Meanwhile, the sample filter 115 may be removed or replaced as needed.

The sample injected into the sample injection region 111 may pass through the sample filter 115 in the opposite direction of gravity and then move to the sample withdrawal region 112. Floating substances or contaminants in the sample may settle to the bottom over time. Accordingly, when the sample injected into the sample injection region 111 passes through the sample filter 115 in the direction of gravity and then moves to the sample withdrawal region 112, some of the micropores of the sample filter 115 may be blocked by the floating substances or contaminants, which may reduce the filtering efficiency. However, when the sample passes through the sample filter 115 in the opposite direction of gravity, the problem of clogging of the micropores may be prevented.

In addition, the sample filter 115 may be installed in a lower portion of the blocking wall 113 to distinguish between the upper portion and the lower portion of the blocking wall 113. For example, the sample filter 115 may be provided in the lower portion of the cylindrical blocking wall 113 to block the inlet of the sample withdrawal region 112. In a case in which the sample filter 115 is installed in the blocking wall 113, the sample filter 115 may be easily replaced by separating the blocking wall 113 from the sample accommodation chamber 110, which has the advantage of facilitating maintenance.

Alternatively, the sample filter 115 may be provided in the sample flow channel 114. Alternatively, the sample filter 115 may be installed in a space between the bottom surface of the sample accommodation chamber 110 and the blocking wall 113.

Meanwhile, the sample flow channel 114 may be provided adjacent to a bottom portion of the sample accommodation chamber 110. In addition, the sample flow channel 114 may be provided at a boundary between the sample injection region 111 and the sample withdrawal region 112. For example, the sample flow channel 114 may be a passage penetrating the blocking wall 113. In detail, the sample flow channel 114 may be a hole penetrating a lower portion of the cylindrical blocking wall 113.

In addition, the sample flow channel 114 may be provided to penetrate both sides of the blocking wall 113. Thus, the flow resistance of the sample flowing through the sample flow channel 114 may be reduced.

In addition, the sample filter 115 may be positioned above the sample flow channel 114. For example, the sample filter 115 may be installed on an inner surface of the cylindrical blocking wall 113, and above the sample flow channel 114.

In addition, the blocking wall 113 may be provided as a separate member from the sample accommodation chamber 110. In addition, the blocking wall 113 may be detachably coupled to the sample accommodation chamber 110. For example, a blocking wall fixing portion may be provided in the sample withdrawal region 112 of the sample accommodation chamber 110 to protrude upward from the bottom surface. In addition, the blocking wall 113 may be coupled to the blocking wall fixing portion to be fixed. For example, the blocking wall 113 may be provided in a cylindrical shape, and the blocking wall fixing portion may be in the shape of a cylinder or a portion of a cylinder for accommodating the blocking wall 113 therein. In addition, the sample flow channel 114 may be formed in the blocking wall 113 and/or the blocking wall fixing portion. For example, the sample flow channel 114 may be a hole penetrating the blocking wall 113 and/or the blocking wall fixing portion.

In addition, the sample accommodation chamber 110 may include a step structure 116 in which the internal cross-sectional area changes drastically between an upper region and a lower region. The step 116 may be provided in the sample injection region 111. For example, the step 116 may be provided on a side surface of the sample accommodation chamber 110 that faces the sample withdrawal region 112.

In addition, an upper wall and a lower wall of the step 116 may include an inclined structure 117. In addition, a surface of the step 116 may have an inclination. Here, the inclination of the stepped surface may be less than the inclinations of an upper surface 117a and a lower surface 117b of the step 116. The inclined structure 117 may guide the injected sample to the bottom of the sample accommodation chamber 110 regardless of the viscosity of the sample.

The lower region of the sample accommodation chamber 110 may correspond to the volume of a single sample. In addition, the upper region of the sample accommodation chamber 110 may correspond to the volume of a plurality of samples for a sample pooling test. For example, when five samples are collected and tested, the samples may fill in the sample accommodation chamber 110 from the lower region to the upper region.

As such, the sample accommodation chamber 110 may include the step structure 116 and the inclined structure 117, so as to have a volume corresponding to a sample pooling test, and secure a sufficient height to allow a sample to be injected even during a single sample test. In a case in which the lower cross-sectional area of the sample accommodation chamber 110 is set to be large, the height of a sample may decrease during a single sample test, resulting an insufficient volume of the sample withdrawn by the pipette tip 400 from the sample withdrawal region 112.

Meanwhile, the sample injection hole 221 of the cover 200 may be positioned above the sample injection region 111 when injecting a sample, and may be positioned above the sample withdrawal region 112 when detecting a sample.

In addition, the cover 200 may be provided such that the sample injection hole 221 and the sample guide portion 222 are positioned above the blocking wall 113 when detecting a sample. For example, the sample guide portion 222 and the blocking wall 113 may be provided in cylindrical shapes with different radii, and the sample guide portion 222 may be provided to have a radius for accommodating the blocking wall 113 therein. Thus, when the cover 200 approaches the chamber body 100 when detecting a sample, the blocking wall 113 may enter the sample guide portion 222 to prevent interference therebetween.

Meanwhile, referring to FIGS. 13 and 14, a bead chamber 120 may accommodate therein beads 122 (or a freeze-dried reagent). In addition, the internal width of the bead chamber 120 may be determined by considering the size of the beads 122. When the internal width of the bead chamber 120 is too large compared to the size of the beads 122, free movement of the beads 122 increases, resulting in a risk that the beads 122 are damaged during packaging and transportation, or stick to the inner wall of the chamber due to frictional static electricity.

In addition, the depth of the bead chamber 120 may be determined by considering the size of the beads 122. When the beads 122 are positioned adjacent to the upper surface of the bead chamber 120, the beads 122 may be affected by heat during a heating process for foil sealing of the bead chamber 120, and thus, the bead chamber 120 may be provided relatively deep compared to the size of the beads 122.

In addition, the width and height of the bead chamber 120 may be determined depending on the size of the beads 122. In detail, the width of the bead chamber 120 may be determined within a range of 150 % to 200 % of the size of the beads 122, and the depth of the bead chamber 120 may be determined within a range of 200 % to 300 % of the size of the beads 122. For example, when the size of the bead is 2.5 mm, the bead chamber 120 may have a circumferential width of 4.5 mm, a radial length of 9.5 mm, and a height of 6.5 mm.

In addition, a guardrail protrusion 121 may be provided on the bottom of a region of the bead chamber 120, to which the pipette tip 400 descends, to prevent the beads 122 from being damaged. The guardrail protrusion 121 may be provided in a shape that does not allow the beads 122 to be seated thereon, and for example, the guardrail protrusion 121 may be provided with a width less than the width of the beads 122, and may be a low-step protrusion extending in the radial direction. This is because, without the guardrail protrusion 121, the beads 122 may be damaged by the tip portion of the pipette tip 400 when the beads 122 are positioned in the region to which the pipette tip 400 descends.

Meanwhile, referring to FIG. 15, the detection chambers 150 having the same shape may be arranged at regular intervals in the direction of the straight line Ld perpendicular to the radial direction Dr of the chamber body 100. In addition, the detection chambers 150 may be in the form of elongated holes extending in directions parallel to the radial direction Dr that is from the center of rotation C toward the center of the detection chambers 150.

For example, in a case in which the detection chambers 150 are provided in an odd number, the detection chambers 150 may be provided in the form of elongated holes extending in directions parallel to the radial direction of the detection chamber 150 located in a central portion among the detection chambers 150. In addition, the detection chambers 150 located on the left and right of the detection chamber 150 located in the central portion may be arranged at regular intervals in the direction of a straight line perpendicular to the radial direction.

As another example, in a case in which the detection chambers 150 are provided in an even number, the detection chambers 150 may be provided in the form of elongated holes extending in directions parallel to the radial direction of the central point between two detection chambers 150 located in the central portion among the detection chambers 150. In addition, the detection chambers 150 located on the left and right of the two detection chambers 150 located in the central portion may be arranged at regular intervals in the direction of a straight line perpendicular to the radial direction.

In addition, referring to FIG. 6, the length of the elongated hole of each of the detection chambers 150 may be set such that, when the pipette tip 400 is positioned above the detection chamber 150, the tip portion of the pipette tip 400 overlaps with the elongated hole. Alternatively, the length of the elongated holes of the detection chambers 150 may be set such that the radial distance Rp from the center of rotation to the tip portion of the pipette tip 400 reaches the interiors of all of the plurality of detection chambers 150. Here, the tip portion of the pipette tip 400 overlaps with the interiors in a plan view, and the tip portion of the pipette tip 400 may be positioned above the detection chambers 150 in a side view.

In addition, in a plan view, the pipette tip 400 may overlap with lower points on radial lines of the elongated holes of one or more detection chambers 150 located in the central portion, and may overlap with upper points on radial lines of the elongated holes of the detection chambers 150 located in other portions than the central portion. That is, the position where the sample is injected from the pipette tip 400 may move from the lower points to the upper points on the radial lines of the plurality of detection chambers 150 as the pipette tip 400 moves from the center to both ends of the line along which the plurality of detection chambers 150 are arranged.

Referring back to FIG. 15, detection regions where samples injected into the respective detection chambers 150 are detected by a photodetector need to be on the same line Ld. That is, an injection region where the sample is injected from the pipette tip 400, and the detection region become farther apart from each other as they move from the center to both sides of the line along which the plurality of detection chambers 150 are arranged, and the injection region and the detection region for the detection chambers 150 located in the central portion may be the same as each other.

In addition, one or more of the plurality of detection chambers 150 may be arranged side by side in the movement direction Ld of the photodetector or in a direction parallel thereto. In addition, one or more of the plurality of detection chambers 150 may be arranged at equal intervals in the movement direction Ld of the photodetector or in a direction parallel thereto.

In addition, the plurality of detection chambers 150 may be in the form of elongated holes extending in directions perpendicular to the movement direction Ld of the photodetector or to a direction parallel thereto. For example, the injection region may be provided at a position close to the elongated hole in the radial direction, and the detection region may be provided at a position far from the elongated hole in the radial direction. However, in some cases, the injection region and the detection region may be provided as one region.

In addition, the detection region may be provided deeper than the injection region. In addition, the injection region may include an inclined portion 151 whose depth increases toward the detection region, and the detection region may include the detection well 152 that is recessed deeper than the inclined portion 151. Thus, the detection chamber 150 may include the detection well 152 in which a nucleic acid is detected, and the inclined portion 151 that guides a sample to the detection well 152. In addition, the detection well 152 may be located farther than the inclined portion 151 in the radial direction. The sample injected into the injection region may move along the inclined portion 151 to be accommodated in the detection well 152. In addition, a boundary between the inclined portion 151 and the detection well 152 is provided with an abrupt discontinuity surface to prevent backflow of the sample.

In addition, a micropattern with a water-repellent function may be formed on the inclined portion 151. For example, the micropattern may be formed with a size of about 100 micrometers. Thus, the sample injected into the inclined portion 151 may flow to the detection well 152 along the micropattern with low frictional resistance, in total.

This is because there may be differences in detection results depending on differences in the volumes of samples accommodated in the respective detection chambers 150. That is, in order to improve detection accuracy, the same volume of sample needs to be accommodated in a plurality of detection wells 152.

In addition, a sample guide protrusion 153 that guides a sample to the detection well 152 may be provided on the inclined portion 151. For example, the sample guide protrusions 153 may be formed on both sides of a region where the sample flows. There may be a portion where the distance between the two sample guide protrusions 153 decreases in the flow direction of the sample.

Meanwhile, beads (not shown) may be accommodated in the detection well 152. For example, freeze-dried beads may be accommodated in the detection well 152. In addition, a bead jamming prevention step (not shown) may be provided in the injection region or between the injection region and the detection region. For example, the bead jamming prevention step may be provided in the inclined portion 151 to limit the movement of the beads to prevent them from being caught on the shallow side of the inclined portion 151. For example, the bead jamming prevention step may be provided in the shape of a protrusion or a rib, and the sample guide protrusion 153 may serve as the bead jamming prevention step.

Meanwhile, the cover 200 may be light-transmissive in part or in whole. The cover 200 may allow excitation light emitted from a light emitter located above the cover 200 to reach the sample accommodated in the detection well 152, and allow light reflected from an optical label of the sample to reach the photodetector. That is, the cover 200 may be provided with light-transmissive paths for the excited light and the reflected light.

Meanwhile, the nucleic acid detection cartridge 10 according to an embodiment of the present disclosure may include a chamber sealing portion capable of sealing one or more of the plurality of chambers. For example, the cover 200 may be equipped with a chamber sealing portion that protrudes downward toward a chamber formed in the chamber body 100, into a shape corresponding to the chamber.

The chamber sealing portion may open the corresponding chamber when the cover 200 is spaced from the chamber body 100, and may be inserted or forcibly fitted into the chamber to hermetically close or seal the chamber when the cover 200 descends to comes into proximity to or into close contact with to the chamber body 100.

In addition, in a case in which a plurality of chambers are arranged side by side, the chamber sealing portions may be arranged side by side according to the shape and arrangement of the plurality of chambers.

As such, by providing the chamber sealing portion, safety and environmental problems that may occur after the use of the nucleic acid detection cartridge 10 may be prevented. When, after the use of the nucleic acid detection cartridge 10, the user detaches the cartridge 10 from a detection device and discards the cartridge 10, reagents, gases, or the like inside the chambers leak out of the cartridge 10, and, as a result, the leakage may harm human safety or health/sanitation and may cause environmental pollution even after the disposal.

However, this risk may be prevented by sealing the chambers with the chamber sealing portion of the nucleic acid detection cartridge 10.

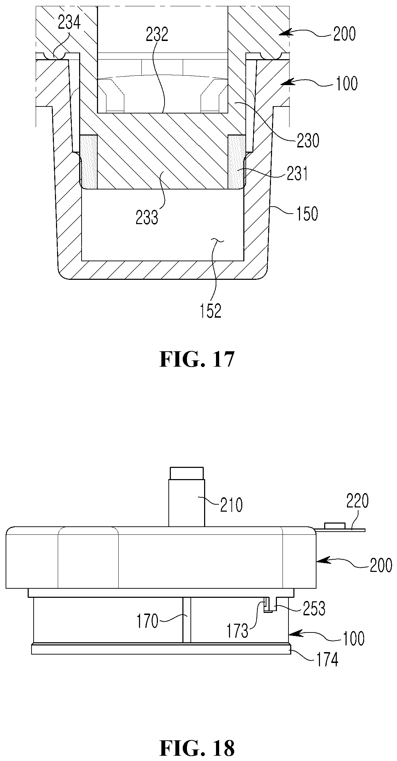

Referring to FIGS. 16 and 17, the cover 200 may further include a detection well sealing portion 230 that seals the detection well 152 of the detection chamber 150. The detection well sealing portion 230 may prevent a sample inside the detection well 152 from leaking out of the detection well 152, and furthermore, may support the pressure that increases as gas is generated while the sample reacts in the detection well 152.

At least one of the chamber body 100 and the cover 200 may be selectively positioned relative to each other in a first position and a second position.

In an embodiment, at least one of the chamber body 100 and the cover 200 may be selectively positioned relative to each other in the first position where the detection well sealing portion 230 opens the detection well 152, and the second position where the detection well sealing portion 230 closes the detection well 152. For example, the cover 200 may be positioned in the first position such that the detection well sealing portion 230 does not seal the detection well 152, and the detection well 152 remains opened. In the extraction step before a nucleic acid detection reaction is performed, the chamber body 100 and the cover 200 may be positioned in the first position. In addition, the cover 200 may be positioned in the second position where the detection well sealing portion 230 seals the detection well 152. While the nucleic acid detection reaction is in progress, the chamber body 100 and the cover 200 may be positioned in the second position.

In addition, at least one of the chamber body 100 and the cover 200 is arranged to be closer to the second position than the first position, relative to each other. In the first position, the detection well sealing portion 230 is spaced from the detection well 152 to open the detection well 152, whereas in the second position, the detection well sealing portion 230 comes into contact with the detection well 152 to seal the detection well 152.In another embodiment, at least one of the chamber body 100 and the cover 200 may be selectively positioned relative to each other in the first position where the sample is withdrawn from the sample accommodation chamber 110 through the sample injection hole 221, and the second position where a nucleic acid is detected in the detection well. For example, the cover 200 may be positioned in the first position where the sample injection hole 221 is located above the sample withdrawal region 112 of the sample accommodation chamber 110. In addition, the cover 200 may be positioned in the second position where the detection well sealing portion 230 is positioned above the detection well 152.

In addition, at least one of the chamber body 100 and the cover 200 is arranged to be closer to the second position than the first position, relative to each other. In the first position, the detection well sealing portion 230 is spaced from the detection well 152 to open the detection well 152, whereas in the second position, the detection well sealing portion 230 comes into contact with the detection well 152 to seal the detection well 152.

In addition, the cover 200 may form the detection well sealing portion 230 that protrudes downward, and the detection well sealing portion 230 may seal the detection well 152 of the detection chamber 150. For example, the detection well 152 may be provided as a cylindrical groove, and the detection well sealing portion 230 may be provided as a cylindrical protrusion to be inserted into the detection well 152. Here, the detection well sealing portion 230 may be a plug to be forcibly fitted into the detection well 152.

In addition, a sealing member 231 may be provided on the outer side surface of the detection well sealing portion 230. The sealing member 231 may be provided on the outer side surface below the protrusion of the detection well sealing portion 230. The sealing member 231 may be made of an elastically deformable material. For example, the sealing member may be formed on the outer side surface of the detection well sealing portion 230 by using a double injection method.

As the cover 200 descends, the detection well sealing portion 230 may be forcibly fitted into the detection well 152 to block the interior of the detection well 152 from the outside. In addition, the detection well sealing portion 230 may be provided to withstand an increase in pressure caused by gas generated inside the detection well 152 due to a frictional force acting between the detection well sealing portion 230 and the inner wall of the detection well 152. Alternatively, by providing downward pressure to the cover 200, it is possible to prevent separation of the detection well sealing portion 230 from the detection well 152 or leakage of gas inside the detection well 152 due to an increase in the pressure inside the detection well 152.

In addition, the cover 200 may further include a detection groove 232 that is recessed from an upper portion of the detection well sealing portion 230. By forming the detection groove 232 in the upper portion of the detection well sealing portion 230, the length of the optical path passing through the cover 200 may be reduced. For example, the detection well sealing portion 230 may be provided in a cylindrical shape having an outer diameter corresponding to the inner diameter of the detection well 152, and the cylindrical detection groove 232 having an inner diameter that is smaller than the outer diameter of the detection well sealing portion 230 may be recessed from the upper portion of the detection well sealing portion 230.

All or part of the detection well sealing portion 230 may include a light-transmitting portion 233. In more detail, all or part of the detection groove 232 may include the light-transmitting portion 233.

The light-transmitting portion 233 may allow excitation light or detection light emitted from a light emitter located above the light-transmitting portion 233 to reach a sample solution accommodated in the detection well 152, and allow light reflected or emitted from the optical label of the sample solution to reach the photodetector. That is, the light-transmitting portion 233 may be provided in paths for the excitation light and the reflected light.

In addition, the cover 200 may further include a periphery sealing portion 234 to be in contact with the upper surface of the chamber body 100 around the detection chamber 150. The periphery sealing portion 234 may be provided on the lower surface of the cover 200 and around the detection well sealing portion 230. In addition, the periphery sealing portion 234 may come into contact with the periphery of the detection chamber 150 to seal the detection chamber 150. For example, the periphery sealing portion 234 may be provided in a ring shape for accommodating the detection chamber 150 therein.

Alternatively, the periphery sealing portion 234 may be formed on the upper surface of the chamber body 100 rather than on the lower surface of the cover 200.

### [Vertical guide structure]

Meanwhile, referring to FIGS. 1 to 9, the cover 200 may be allowed to move downward only at a certain position in the chamber body 100.

The cover 200 is rotatable while being spaced from the upper surface of the chamber body 100 during a sample preparation step before detecting a target material, but during a target material detection step, the detection well sealing portion 230 descends to the proximity of the upper surface of the chamber body 100 while being aligned above the detection well 152, such that the detection well sealing portion 230 seals the detection well 152.

However, when the cover 200 descends toward the chamber body 100 while the detection well sealing portion 230 is not aligned above the detection well 152, interference occurs between the cover 200 and the chamber body 100.

To address this issue, the cover 200 and the chamber body 100 may include a vertical guide structure. The vertical guide structure may include protrusions and grooves. By the protrusions and the grooves, the chamber body 100 or the cover 200 may move in a vertical direction, that is, in the direction of the axis of rotation, at a position where the detection well sealing portion 230 and the detection well 152 are aligned. Thus, the vertical guide structure allows the chamber body 100 or the cover 200 to move in the direction of the axis of rotation at a predetermined position in the circumferential direction.

The cover 200 may have a vertical guide protrusion 250 on the inner side surface thereof, and the chamber body 100 may have, on the outer side surface thereof, a vertical guide groove 170 extending in the vertical direction to guide the vertical guide protrusion 250 in the vertical direction.

Conversely, the chamber body 100 may have a vertical guide protrusion on the outer side surface thereof, and the cover 200 may have, on the inner side surface thereof, a vertical guide groove extending in the vertical direction to guide the vertical guide protrusion of the chamber body 100 in the vertical direction.

Alternatively, the vertical guide structure may include a protrusion formed on the lower surface of the cover 200, and a groove formed on the upper surface of the chamber body 100. In this case, the cover 200 may be allowed to descend only when the protrusion and the groove are aligned with each other as the cover 200 rotates.

In addition, two or more vertical guide structures may be provided in the rotational direction or the circumferential direction. Therefore, distortion may be prevented during a vertical movement, and durability may be improved. For example, four vertical guide structures may be provided at 90-degree intervals in the direction of rotation, or three vertical guide structures may be provided at 120-degree intervals.

In addition, a plurality of vertical guide structures may be provided at different intervals. For example, three vertical guide structures may be provided at 130-degree and 110-degree intervals, respectively. In this case, the above alignment of the vertical guide structures may be proper at only one position. Thus, the cover 200 may be allowed to descend only when the detection well sealing portion 230 is aligned above the detection well 152 in the target material detection step.

### [Rotation prevention structure]

Meanwhile, it is necessary to prevent the user from arbitrarily rotating the cover 200 before the nucleic acid detection cartridge 10 is mounted on a detection device.

In the initial state of the product, the vertical guide structure may have been misaligned. Thus, it is possible to prevent the user from arbitrarily pressing the cover. However, it is necessary to prevent the user from arbitrarily rotating the cover to align the vertical guide structure.

In addition, in the initial state of the cartridge 10, the pipette tip 400 may be provided as being accommodated in the space of the tip mounting chamber 142, and in this case, when the user arbitrarily rotates the cover 200, damage to the pipette tip 400 may occur.

In addition, when the cover 200 is allowed to rotate arbitrarily before the cartridge 10 is inserted into the detection device, the cover 200 at the point where the vertical guide structure matches is allowed to descend toward the chamber body 100, which may cause damage to the sealing film 300 that seals the chambers of the chamber body 100.

To address this issue, the cover 200 and the chamber body 100 may include a rotation prevention structure. The rotation prevention structure may include protrusions and grooves.

Referring to FIG. 18, the cover 200 may have a rotation prevention protrusion 253 below the inner rear surface thereof, and the chamber body 100 may have an rotation prevention groove 173 on the outer rear surface thereof to accommodate the rotation prevention protrusion 253. Conversely, the chamber body 100 may have an rotation prevention protrusion on the outer rear surface thereof, and the cover 200 may have an rotation prevention groove on the inner rear surface thereof to that accommodate the rotation prevention protrusion.

In addition, the rotation prevention protrusion 253 may be provided to be elastically deformable in the radial direction. For example, the rotation prevention protrusion 253 may have a cantilever shape extending downward from the cover 200. Alternatively, cut-out portions may be formed on both sides of the rotation prevention protrusion 253. Thus, when the cover 200 is rotated with a certain torque or greater, the rotation prevention protrusion 253 may move outward in the radial direction to be separated from the rotation prevention groove 173. After the rotation prevention protrusion 253 is separated from the rotation prevention groove 173, the cover 200 may be rotatable above the chamber body 100.

### [Press prevention structure]

Meanwhile, the cover 200 may be prevented from moving downward toward the chamber body 100 under a certain pressure or lower. This is because, the cover 200 may be allowed to descend toward the chamber body 100 at the point where the vertical guide structure is aligned in the sample preparation step, which may cause damage to the sealing film 300 that seals the chambers of the chamber body 100.

To address this issue, the cover 200 and the chamber body 100 may include a press prevention structure. The press prevention structure may include protrusions and catching jaws.

For example, the cover 200 may be provided with a press prevention protrusion (not shown) that protrudes outward in the radial direction from a lower portion of the connecting rod 240, and may be provided with a press prevention jaw (not shown) that protrudes inward in the radial direction from an upper portion of the connecting groove 162 of the chamber body 100.

In addition, the press prevention protrusion may be provided to be elastically deformable in the radial direction. For example, the press prevention protrusion may have a cantilever shape extending downward from the cover 200. Alternatively, cut-out portions may be formed on both sides of the press prevention protrusion. Thus, when the cover 200 is pressed with a certain pressure or higher, the press prevention protrusion moves inward in the radial direction to be separated from the press prevention jaw. After the press prevention protrusion is separated from the press prevention jaw, the cover 200 may move downward toward the chamber body 100.

### [Separation prevention structure]

Meanwhile, the cover 200 may be prevented from being separated upward from the chamber body 100. This is because, when the user is allowed to arbitrarily separate the cover 200 from the chamber body 100, the sealing film 300 that seals the chambers of the chamber body 100 may be damaged.

To address this issue, the cover 200 and the chamber body 100 may include a separation prevention structure. The separation prevention structure may include protrusions and catching jaws.

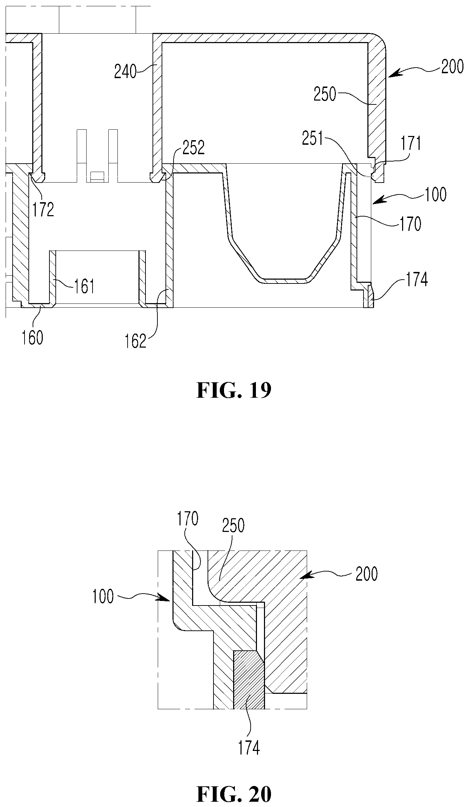

Referring to FIG. 19, the cover 200 may be provided with a first separation prevention protrusion 251 that protrudes inward in the radial direction from a lower portion of the inner side surface thereof, and the chamber body 100 may be provided with a first separation prevention jaw 171 that protrudes outward in the radial direction from an upper portion of the outer side surface thereof.

The cover 200 may be restricted from moving upward by the first separation prevention protrusion 251 being caught by the first separation prevention jaw 171, but the first separation prevention protrusion 251 may be allowed to move downward along the outer side surface of the chamber body 100.

In addition, the cover 200 may be provided with a second separation prevention protrusion 252 that protrudes outward in the radial direction from a lower portion of the connecting rod 240, and the chamber body 100 may be provided with a second separation prevention jaw 172 that protrudes inward in the radial direction from an upper portion of the connecting groove 162.

The cover 200 may be restricted from moving upward by the second separation prevention protrusion 252 being caught by the second separation prevention jaw 172, but the second separation prevention protrusion 252 may be allowed to move downward along the inner side surface of the connecting groove 162.

### [Sealing ring]

Meanwhile, referring to FIGS. 19 and 20, the cover 200 or the chamber body 100 may further include a sealing ring 174 that blocks the chambers of the chamber body 100 from outside air.

For example, the sealing ring 174 may be provided on the outer side surface of the chamber body 100, in a ring shape that surrounds the chamber body 100 in the circumferential direction. In addition, the sealing ring 174 may block outside air from the cartridge 10 while in contact with the inner side surface of the cover 200.

Alternatively, the sealing ring 174 may be provided on the inner side surface of the cover 200, in a ring shape that surrounds the chamber body 100 in the circumferential direction. In addition, the sealing ring 174 may block outside air from the cartridge 10 while in contact with the outer side surface of the chamber body 100.

In addition, the sealing ring 174 may be positioned in a lower portion of the chamber body 100, to be lower than the vertical guide groove 170. Because the sealing ring 174 is positioned lower than the vertical guide groove 170, the sealing ring 174 may be provided in a continuous ring shape on the outer side surface of the chamber body 100.

In a sample preparation step, when the cover 200 is spaced from the upper surface of the chamber body 100, the sealing ring 174 may be provided without contacting the inner side surface of the cover 200. Thus, the cover 200 or the chamber body 100 may be allowed to rotate freely.

In addition, in a nucleic acid detection step, when the cover 200 comes into proximity to or into close contact with the chamber body 100, the sealing ring 174 may be in contact with the inner side surface of the cover 200. Thus, the cover 200 may block outside air from the cartridge 10.

Alternatively, the sealing ring 174 may be provided in an upper portion of the outer side surface of the chamber body 100 or in a lower portion of the inner side surface of the cover 200. In this case, the sealing ring 174 may always be interposed between the chamber body 100 and the cover 200 regardless of the position of the cover 200. Here, the sealing ring 174 may have a coefficient of friction sufficient to allow free rotation of the chamber body 100 or the cover 200.

In addition, the sealing ring 174 may be provided as a separate member and then coupled to the cover 200 or the chamber body 100, or may be formed in the cover 200 or the chamber body 100 by using a double injection molding method.

Next, a method of using a nucleic acid detection device and the nucleic acid detection cartridge 10 according to an embodiment of the present disclosure will be described.

The nucleic acid detection cartridge 10 is provided in a packaged state. The user unpacks and takes out the nucleic acid detection cartridge 10. Then, after opening the stopper portion 220 of the cover 200, a sample may be injected through the sample injection hole 221 by using a pipette. In the initial state, the sample injection hole 221 is located above the sample injection region 111 of the sample accommodation chamber 110, and thus, the sample is injected into the sample injection region 111 in total. After injecting the sample, the user closes the stopper portion 220 of the cover 200.

Then, the user mounts the cartridge 10 into the nucleic acid detection device. For example, a robotic arm of the nucleic acid detection device may move to the outside of the device, and the user may place the nucleic acid detection device on the robotic arm. Alternatively, the user may mount the cartridge 10 by inserting it into a slot of the nucleic acid detection device.

At this time, the cartridge 10 may be mounted such that the cover 200 is positioned above and the chamber body 100 is positioned below.

Thereafter, the robotic arm moves into the device such that the center of the cartridge 10 is aligned with the drive shaft of the rotation driving unit. Then, the cartridge 10 or the drive shaft is moved to couple the cartridge 10 to the drive shaft. For example, as the robotic arm puts down the cartridge 10, the drive shaft may be inserted into and coupled to the collar portion 161 of the coupling mounting portion 160.

Next, the detection device moves the pipette to couple an end portion of the pipette to the pipette tip 400. In the initial state, the pipette tip 400 of the cartridge 10 is positioned above the tip mounting chamber 142. The detection device may restrain the cover 200 from rotating during the process of coupling the pipette to the pipette tip 400. For example, as the detection device presses the pipette tip 400 by moving the pipette downward, the first catching protrusion 405 of the pipette tip 400 is supported by the support jaw 212 of the pipette tip guide 210, and in this state, pressure is applied to the pipette tip 400 to be coupled. In addition, before the chamber body 100 rotates, the pipette is raised such that the tip portion of the pipette tip 400 is positioned higher than the upper surface of the chamber body 100.

Meanwhile, referring to FIG. 6, the tip mounting chamber 142 may be provided in the form of an elongated hole extending in the circumferential direction. The tip mounting chamber 142 is provided to accommodate the tip portion 403 when the cartridge 10 is in the initial state, i.e., in the position where a sample is injected, and to accommodate the tip portion 403 when the cartridge 10 is in a detection state. For example, the position of the tip portion 403 when a sample is injected and the position of the tip portion 403 when a nucleic acid is detected may be arranged to be offset by a certain angle, at the same radial distance from the center of rotation C.

Thereafter, the driving unit rotates the chamber body 100 in one direction by a preset angle. When the chamber body 100 rotates by a certain angle, the tip portion of the pipette tip 400 is positioned above the sample withdrawal region 112 of the sample accommodation chamber 110. Then, the pipette is moved downward such that the tip portion of the pipette tip 400 enters the space defined by the blocking wall 113 to be immersed in the sample, and then negative pressure is applied to the pipette to withdraw the sample. At this time, the sample being withdrawn has been refined while passing through the sample filter 115.

Thereafter, the driving unit rotates the chamber body 100 in one direction by a preset angle. When the chamber body 100 rotates by a certain angle, the tip portion of the pipette tip 400 is positioned above a target chamber. Then, the pipette moves downward such that the tip portion 403 penetrates the sealing film 300 to enter the target chamber. Then, a sample or a reagent may be aspirated by applying negative pressure to the pipette, or a sample or a reagent may be injected by applying positive pressure to the pipette.

As the chamber body 100 rotates, the pipette tip 400 may be positioned above the target chamber, and a series of sample preparation steps may be performed while the pipette tip 400 aspirates and injects samples or reagents. For example, the pipette may aspirate a buffer from one buffer chamber 130, and inject a buffer into one bead chamber 120. In addition, when the beads are dissolved in the buffer, the pipette may aspirate them again and then inject them into the mixing chamber 141. By repeating this series of processes, the sample preparation step is completed.

In addition, the detection device may include a heating element capable of transferring heat to any one of the chambers in this process. For example, the heating unit of the detection device may transfer heat to the bead chamber 120 to promote dissolution of the beads, transfer heat to the mixing chamber 141 to promote mixing of the sample and the reagent, and transfer heat to the detection well 152 to cause a detection reaction.

Thereafter, the driving unit rotates the chamber body 100 in one direction by a preset angle. When the chamber body 100 rotates by a certain angle, the tip portion of the pipette tip 400 may be positioned above the injection region of the detection chamber 150. Then, the pipette moves downward such that the tip portion 403 penetrates the sealing film 300 to enter the detection chamber 150. Then, positive pressure is applied to the pipette to eject the sample into the injection region. The sample moves from the injection region to the detection region along the inclined portion 151, and is consequently accommodated in the detection chamber 150.

Here, the injection region and the detection region of the detection chamber 150 may be different from or identical to each other. In addition, the injection region of the detection chamber 150 may be the inclined portion 151 or the detection well 152. When the detection chamber 150 directly injects a sample into the detection well 152, the injection region and the detection region may refer to the same region.

Thereafter, the driving unit rotates the chamber body 100 in one direction by a preset angle. When the chamber body 100 rotates by a certain angle, the detection well sealing portion 230 may be positioned above the detection well 152. In this state, the vertical guide structure is aligned such that the cover 200 may be allowed to move downward toward the chamber body 100. In detail, the vertical guide protrusion 250 may be positioned above the vertical guide groove.

Thereafter, the detection device presses down the cover 200 by applying a certain pressure or higher. Then, the detection well sealing portion 230 of the cover 200 ruptures the sealing film 300 and then is settled on an inner wall of the detection well 152. At this time, in a case in which the press prevention structure is applied to the cartridge 10, the press prevention protrusion is deformed by pressure to pass through the press prevention jaw, and the cover 200 may move downward.

In addition, the detection device may provide additional pressure to the cover 200 to maintain the state in which the detection well sealing portion 230 applies pressure to the detection well 152. When a detection reaction occurs as heat is applied to the detection well 152, gas is generated, and the pressure inside the detection well 152 may increase due to the generated gas. The detection device may apply a force to press the cover 200 to prevent gas inside the detection well 152 from leaking out the detection well 152.

In addition, the detection device drives the detection module in the direction of arrangement of the detection chambers 150. That is, the detection module detects a plurality of detection chambers 150 while moving along a straight line. At this time, the detection module is in a stationary state, and even when the cartridge 10 rotates in one direction, the detection module may detect the plurality of detection chambers 150. However, a fluid flow occurs inside the detection well 152 due to the centrifugal force of the cartridge 10, and as a result, it may be difficult to expect accurate detection results.

The present application claims priority to Korean Patent Application Nos. 10-2022-0006173, 10-2022-0006174, 10-2022-0006175, and 10-2022-0006175, filed on May 6, 2022 in Korea, the disclosures of which are incorporated by reference herein in their entireties.

The above description merely explains the idea of the present disclosure and the present disclosure may be changed and modified in various ways without departing from the scope of the present disclosure by those of skill in the art. Accordingly, the embodiments described herein are provided not to limit, but to merely explain the idea of the present disclosure, and the idea of the present disclosure is not limited by the embodiments. The scope of the present disclosure should be construed by the following claims, and all technical ideas within the equivalent scope should be construed as being included in the scope of the present disclosure.

### EXPLANATION OF REFERENCE NUMERALS

| | | | |
|---|---|---|---|
| 10: | Nucleic acid detection cartridge, | | |
| 100: | Chamber body, | | |
| 110: | Sample accommodation chamber, | 111: | Sample injection region, |
| 112: | Sample withdrawal region, | 113: | Blocking wall, |
| 114: | Sample flow channel, | 115: | Sample filter, |
| 116: | Step, | 117: | Inclined structure |
| 120: | Bead chamber, | 121: | Guardrail protrusion, |
| 122: | Beads, | 130: | Buffer chamber, |
| 141: | Mixing chamber, | 142: | Tip mounting chamber, |
| 143: | Dummy chamber, | 150: | Detection chamber, |
| 151: | Inclined portion, | 152: | Detection well, |
| 153: | Sample guide protrusion, | 160: | Mounting portion, |
| 161: | Collar portion, | 162: | Connecting groove, |
| 170: | Vertical guide groove, | | |
| 171: | First separation prevention jaw, | | |
| 172: | Second separation prevention jaw, | | |
| 173: | Rotation prevention groove, | | |
| 174: | Sealing ring, | 200: | Cover, |
| 210: | Pipette tip guide, | 211: | Pipette hole, |
| 212: | Support jaw, | 220: | Stopper portion, |
| 221: | Sample injection hole, | 222: | Sample guide portion, |
| 230: | Detection well sealing portion, | | |
| 231: | Sealing member, | 232: | Detection groove, |
| 233: | Light-transmitting portion, | 234: | Periphery sealing portion, |
| 240: | Connecting rod, | 250: | Vertical guide protrusion, |
| 251: | First separation prevention protrusion, | | |
| 252: | Second separation prevention protrusion, | | |
| 253: | Rotation prevention protrusion, | | |
| 300: | Sealing film, | 400: | Pipette tip, |
| 401: | Connecting portion, | 402: | Pipette tip body, |
| 403: | Tip portion, | 404: | Pipette tip filter member, |
| 405: | First catching protrusion, | 406: | Second catching protrusion. |

## Claims

1. A nucleic acid detection cartridge, comprising:
a chamber body including a plurality of chambers provided on the upper surface; and
a cover facing the upper surface of the chamber body,
wherein at least one of the chamber body and the cover can move relative to the other in a plane direction parallel to the upper surface, and
wherein at least one of the chamber body and the cover can move relative to the other in a straight line a certain angle with respect to the upper surface.

2. The nucleic acid detection cartridge of claim 1, wherein the plurality of chambers comprise chambers arranged in a circumferential direction with respect to a center of the chamber body, and
wherein the cover is configured to cover the chambers.

3. The nucleic acid detection cartridge of claim 1, wherein at least one of the chamber body and the cover is configured to be rotationally movable relative to each other about an axis of rotation, and
wherein at least one of the chamber body and the cover is configured to translate relative to each other in a direction of the axis of rotation.

4. The nucleic acid detection cartridge of claim 1, wherein the cover comprises a pipette hole provided on a movement path of a pipette or a pipette tip that aspirates fluids from one or more of the plurality of chambers and injects fluids into one or more of the plurality of chambers.

5. The nucleic acid detection cartridge of claim 4, further comprising the pipette tip coupled to an end portion of the pipette, and configured to be vertically movable through the pipette hole of the cover.

6. The nucleic acid detection cartridge of claim 5, wherein the cover comprises a pipette tip guide configured to extend upward from the pipette hole and accommodate the pipette tip therein.

7. The nucleic acid detection cartridge of claim 5, wherein the pipette tip comprises a connecting portion configured to couple pipette, a pipette tip body extending downward from the connecting portion to form a flow path, a tip portion extending downward from the pipette tip body, and a pipette tip filter member provided in the flow path inside the pipette tip body.

8. The nucleic acid detection cartridge of claim 3, wherein the chamber body is configured to rotate about a central axis,
wherein the plurality of chambers comprise chambers arranged in the circumferential direction with respect to a center of the chamber body.

9. The nucleic acid detection cartridge of claim 8,
wherein the chamber body is provided in a shape of a circular disk or a shape of a portion of a disk, and
wherein the cover comprises an inner side surface facing an outer side surface of the chamber body, and an upper portion facing the upper surface of the chamber body.

10. The nucleic acid detection cartridge of claim 9, further comprising a sealing ring that is provided on the inner side surface of the cover or on the outer side surface of the chamber body, and blocks air outside the cover.

11. The nucleic acid detection cartridge of claim 10, wherein the sealing ring is configured in a lower portion of the outer side surface of the chamber body, when the cover is relatively distant from the chamber body in the direction of the axis of rotation, the sealing ring is not in contact with the cover, and when the cover is relatively close to the chamber body in the direction of the axis of rotation, the sealing ring comes into contact with the cover.

12. The nucleic acid detection cartridge of claim 10, wherein the sealing ring has a coefficient of friction that allows relative rotation of the cover and the chamber body.

13. The nucleic acid detection cartridge of claim 5, further comprising a sealing film that is attached or coupled to the upper surface of the chamber body to seal the plurality of chambers.

14. The nucleic acid detection cartridge of claim 13, wherein the pipette tip is configured to descend to penetrate the sealing film.

15. The nucleic acid detection cartridge of claim 9, wherein vertical guide structures are provided on the inner side surface of the cover and the outer side surface of the chamber body, respectively, to guide a vertical movement of the chamber body or the cover, and
wherein the vertical guide structures allow the chamber body or the cover to move in the direction of the axis of rotation at a predetermined position in the circumferential direction.

16. The nucleic acid detection cartridge of claim 9, further comprising vertical guide structures that guide a vertical movement of the chamber body or the cover,
wherein the vertical guide structures are provided on the upper surface of the chamber body and a bottom surface of the cover that faces the upper surface of the chamber body, respectively, and
wherein the vertical guide structures allow the chamber body or the cover to move in the direction of the axis of rotation at a predetermined position in the circumferential direction.

17. The nucleic acid detection cartridge of claim 1, wherein the chamber body further comprises a sample accommodation chamber into which a sample is injected, and
wherein the cover comprises a sample injection hole configured to, when aligned with the sample accommodation chamber, open the sample accommodation chamber.

18. The nucleic acid detection cartridge of claim 17, wherein the sample accommodation chamber is configured to be spatially partitioned into a sample injection region where the sample is injected, and a sample withdrawal region where the sample is withdrawn, and
wherein the sample accommodation chamber comprises a filter member that the sample, injected into the sample injection region, passes through before being withdrawn at the sample withdrawal region.

19. The nucleic acid detection cartridge of claim 18, wherein the sample injection hole is configured to be positioned above the sample injection region when the sample is injected, and be positioned above the sample withdrawal region when a nucleic acid is detected.

20. The nucleic acid detection cartridge of claim 19, wherein the cover comprises a sample guide portion that extends toward a lower side of the sample injection hole and is configured to be accommodated in the sample withdrawal region as the chamber body and the cover come into proximity to each other.

21. The nucleic acid detection cartridge of claim 1, wherein the chamber body further comprises a detection chamber in which a nucleic acid is detected, and
wherein the cover comprises a light-transmitting portion which transmits detection light from the detection chamber.

22. The nucleic acid detection cartridge of claim 21, wherein the detection chamber comprises a detection well in which the nucleic acid is detected,
wherein the cover comprises a detection well sealing portion configured to seal the detection well, and
wherein at least one of the chamber body and the cover is configured to be selectively positioned relative to each other in a first position where the detection well sealing portion opens the detection well and a second position where the detection well sealing portion closes the detection well.

23. A nucleic acid detection cartridge, comprising:
a chamber body comprising a sample accommodation chamber into which a sample is injected, and a detection chamber comprising a detection well in which a nucleic acid is detected; and
a cover having an opening capable of opening the sample accommodation chamber, and a light-transmitting portion capable of transmitting detection light into the detection well,
wherein at least one of the chamber body and the cover is configured to be selectively positioned relative to each other in a first position where the sample is withdrawn from the sample accommodation chamber through the opening, and a second position where the nucleic acid is detected in the detection well, and
wherein at least one of the chamber body and the cover is configured to be closer to the second position than the first position, relative to each other.

24. A nucleic acid detection cartridge, comprising:
a chamber body comprising a plurality of chambers provided on an upper surface thereof;
a cover facing the upper surface of the chamber body; and
a pipette tip configured to be vertically movable relative to the cover, and to be coupled to an end portion of a pipette,
wherein at least one of the chamber body and the cover is configured to be rotatable relative to each other, and
wherein the pipette tip transfers fluids between the plurality of chambers while changing a relative position thereof in a circumferential direction of the chamber body together with the cover.

25. The nucleic acid detection cartridge of claim 24, wherein the cover comprises a pipette hole provided on a movement path of the pipette tip.

26. The nucleic acid detection cartridge of claim 25, wherein the cover comprises a pipette tip guide that extends upward from the pipette hole and accommodates the pipette tip therein.

27. The nucleic acid detection cartridge of claim 26, wherein the pipette tip comprises a connecting portion to which the pipette is able to be coupled, a pipette tip body extending downward from the connecting portion to form a flow path, a tip portion extending downward from the pipette tip body, and a pipette tip filter member provided in the flow path inside the pipette tip body.

28. The nucleic acid detection cartridge of claim 27, wherein the connecting portion has a first catching protrusion that extends toward an outside of an opening of the pipette tip guide such that the pipette tip is seated on the pipette tip guide.

29. The nucleic acid detection cartridge of claim 27, wherein the pipette tip body has a second catching protrusion that extends toward an outside of an opening of the pipette tip guide to prevent the pipette tip from being separated upward from the cover.

30. The nucleic acid detection cartridge of claim 29, wherein the second catching protrusion is provided to protrude in a circumferential direction of the pipette tip body, and to be elastically deformable.

31. The nucleic acid detection cartridge of claim 30, wherein the pipette tip guide comprises a step structure in which an upper inner diameter is less than a lower inner diameter, and
wherein the second catching protrusion has an outer diameter greater than the upper inner diameter of the pipette tip guide.

32. The nucleic acid detection cartridge of claim 24, further comprising a sealing film that is attached or coupled to the upper surface of the chamber body to seal the plurality of chambers.

33. The nucleic acid detection cartridge of claim 32, wherein the sealing film is configured to be penetrated by the pipette tip.

34. The nucleic acid detection cartridge of claim 24, wherein the plurality of chambers comprise chambers arranged in the circumferential direction with respect to a center of the chamber body, and
wherein the cover is configured to cover the chambers.

35. The nucleic acid detection cartridge of claim 34, wherein at least one of the chamber body and the cover is configured to be rotationally movable relative to each other.

36. The nucleic acid detection cartridge of claim 35, wherein at least one of the chamber body and the cover is configured to be able to translate relative to each other in a direction of the axis of rotation.

37. The nucleic acid detection cartridge of claim 34, wherein the chamber body comprises a plurality of chambers arranged in the circumferential direction with respect to a center, and detection chambers arranged side by side, and
wherein the detection chambers are arranged such that a straight line having a certain angle to any one radial direction of the chamber body passes through the detection chambers.

38. The nucleic acid detection cartridge of claim 37, wherein the detection chambers are arranged such that the pipette performing a relative circular motion at a certain radius away from a central axis of the chamber body passes through the detection chambers.

39. The nucleic acid detection cartridge of claim 38, wherein the detection chamber comprises a detection well in which a nucleic acid is detected, and an inclined portion that guides a sample to the detection well.

40. The nucleic acid detection cartridge of claim 39, wherein the detection chamber is provided in a form of an elongated hole extending in a direction parallel to the radial direction, and
wherein the detection well is positioned farther away in the radial direction than the inclined portion.

41. The nucleic acid detection cartridge of claim 39, wherein the cover comprises a detection well sealing portion capable of sealing the detection well, and
wherein at least one of the chamber body and the cover is able to be selectively positioned relative to each other in a first position where the detection well sealing portion opens the detection well, and a second position where the detection well sealing portion seals the detection well.

42. The nucleic acid detection cartridge of claim 41, wherein the cover comprises a light-transmitting portion capable of transmitting detection light into the detection well.

43. The nucleic acid detection cartridge of claim 1, wherein the chamber body further comprises a sample accommodation chamber into which a sample is injected,
wherein the sample accommodation chamber comprises a sample injection region into which the sample is injected, a sample withdrawal region that is spatially separated from the sample injection region, and from which the sample is withdrawn, and a filter member, and
wherein the sample injected into the sample injection region passes through the filter member in a direction opposite to gravity, and then moves to the sample withdrawal region.

44. The nucleic acid detection cartridge of claim 1, wherein the chamber body comprises the plurality of chambers comprising a sample accommodation chamber in which a sample is accommodated, and detection chambers each comprising a detection well in which a nucleic acid is detected,
wherein the cover comprises a detection well sealing portion capable of sealing the detection well, and
wherein at least one of the chamber body and the cover is able to be selectively positioned relative to each other in a first position where the detection well sealing portion opens the corresponding detection well, and a second position where the detection well sealing portion seals the corresponding detection well.

45. The nucleic acid detection cartridge of claim 1, wherein the chamber body is provided in a shape of a circular disk or a shape of a portion of a disk, and comprises the plurality of chambers that are arranged in a circumferential direction with respect to a central axis, and comprise a sample accommodation chamber, and detection chambers in which nucleic acids are detected, and
wherein the detection chambers are arranged side by side such that a straight line having a certain angle to any one radial direction of the chamber body passes through the detection chambers.

46. A nucleic acid detection cartridge, comprising a chamber body comprising a sample accommodation chamber in which a sample is accommodated,
wherein the sample accommodation chamber comprises a sample injection region into which the sample is injected, a sample withdrawal region that is spatially separated from the sample injection region, and from which the sample is withdrawn, and a filter member, and
wherein the sample injected into the sample injection region passes through the filter member in a direction opposite to gravity, and then moves to the sample withdrawal region.

47. A nucleic acid detection cartridge, comprising:
a chamber body comprising a sample accommodation chamber in which a sample is accommodated, and detection chambers each comprising a detection well in which a nucleic acid is detected; and
a cover that is configured to cover an upper surface of the chamber body, and comprises a detection well sealing portion capable of sealing the detection well,
wherein at least one of the chamber body and the cover is able to be selectively positioned relative to each other in a first position where the detection well sealing portion opens the corresponding detection well, and a second position where the detection well sealing portion seals the corresponding detection well.

48. A nucleic acid detection cartridge, comprising a chamber body that is provided in a shape of a circular disk or a shape of a portion of a disk, and comprises a plurality of chambers that are arranged in a circumferential direction with respect to a central axis, and comprise a sample accommodation chamber, and detection chambers in which nucleic acids are detected,
wherein the detection chambers are arranged side by side such that a straight line having a certain angle to any one radial direction of the chamber body passes through the detection chambers.
